Europäisches Patentamt
European Patent Office
Office européen des brevets

(11) Veröffentlichungsnummer: **0 419 410 A2**

# EUROPÄISCHE PATENTANMELDUNG

(21) Anmeldenummer: **90810685.9**

(22) Anmeldetag: **11.09.90**

(51) Int. Cl.5: **C07D 311/24**, C07D 405/04, A61K 31/35, C07D 407/12, C07D 409/12, C07D 407/14, C07D 409/14

(30) Priorität: **19.09.89 CH 3401/89**

(43) Veröffentlichungstag der Anmeldung: **27.03.91 Patentblatt 91/13**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **von Sprecher, Andreas, Dr.**
**Nelkenweg 1**
**CH-4104 Oberwil(CH)**
Erfinder: **Beck, Andreas, Dr.**
**Reutebachgasse 40**
**W-7800 Freiburg(DE)**

(54) **Weitere neue Alkanophenone.**

(57) Substituierte Alkanophenone der allgemeinen Formel

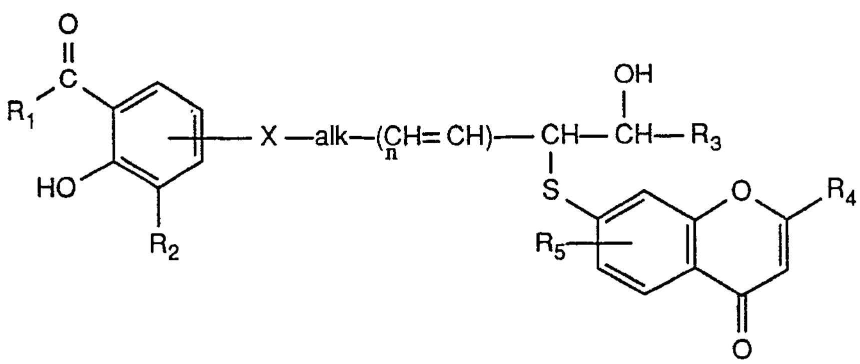

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ einen unsubstituierten oder durch gegebenenfalls fluoriertes Niederalkyl, veräthertes oder verestertes Hydroxy, Halogen, gegebenenfalls niederalkyliertes Amino und/oder gegebenenfalls verestertes oder amidiertes Carboxy substituierten 5-gliedrigen, als Heteroatom 1 N-, O- oder S-Atom aufweisenden Heteroarylrest bedeutet, $R_4$ gegebenenfalls verestertes oder amidiertes Carboxy oder 5-Tetrazolyl darstellt und $R_5$ für Wasserstoff oder Niederalkyl steht, haben Leukotrien-antagonistische Eigenschaften und können als antiallergische Arzneimittelwirkstoffe verwendet werden. Das Verfahren zu ihrer Herstellung ist dadurch gekennzeichnet, dass man ein Epoxid der Formel II

EP 0 419 410 A2

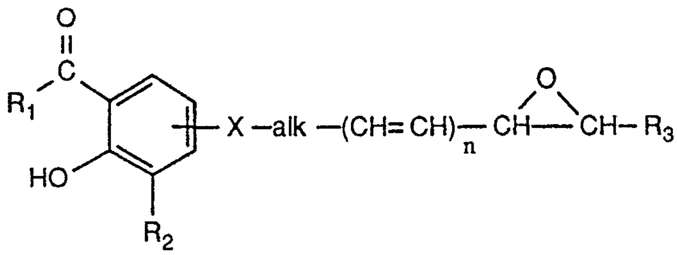

(II),

worin $R_1$, $R_2$, X, alk, n und $R_3$ obige Bedeutungen haben, mit einem Thiol der Formel III

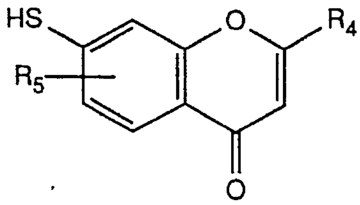

(III),

worin $R_4$ und $R_5$ obige Bedeutungen haben, oder einem Salze davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältiches Salz in die freie Verbindung oder in ein anderes Salz überführt.

## WEITERE NEUE ALKANOPHENONE

Die Erfindung betrifft neue substituierte Alkanophenone der allgemeinen Formel I

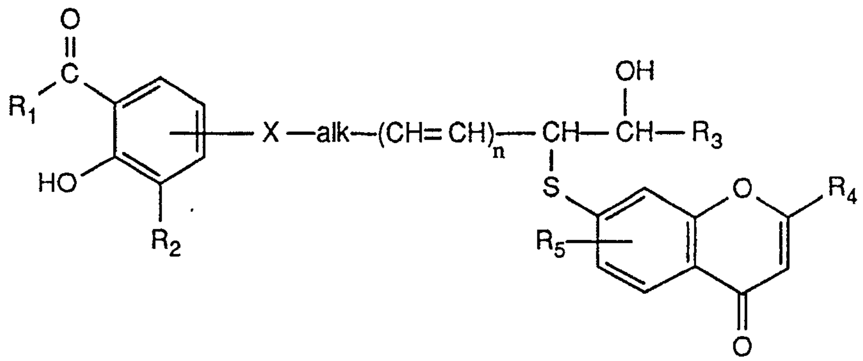

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ einen unsubstituierten oder durch gegebenenfalls fluoriertes Niederalkyl, veräthertes oder verestertes Hydroxy, Halogen, gegebenenfalls niederalkyliertes Amino und/oder gegebenenfalls verestertes oder amidiertes Carboxy substituierten 5-gliedrigen, als Heteroatom 1 N-, O- oder S-Atom aufweisenden Heteroarylrest bedeutet, $R_4$ gegebenenfalls verestertes oder amidiertes Carboxy oder 5-Tetrazolyl darstellt und $R_5$ für Wasserstoff oder Niederalkyl steht, und ihre Salze, Verfahren zu ihrer Herstellung, sie als Wirkstoff enthaltende pharmazeutische Präparate und ihre Verwendung als Arzneimittelwirkstoffe.

Die Raumdarstellung in der obigen Formel I ist für die bevorzugten Verbindungen, in welchen das O-Atom der Hydroxygruppe mit dem S-Atom in der relativen trans -Konfiguration sind, so zu verstehen, dass die Symbole der ersten Zeile oberhalb, die der dritten Zeile dann unterhalb der Darstellungsebene liegen (oder umgekehrt), was für die abgebildete Formel der entgegengesetzten Konfiguration, (RS)-(SR), gemäss der Kahn-Ingold-Prelog-Konvention an dem mit dem Schwefelatom verbundenen Kohlenstoffatom, (C-S-), und dem die Hydroxygruppe tragenden Kohlenstoffatom, (C-OH), entspricht. Dabei sind, wenn n für 2 steht, die Enantiomeren mit S(C-S-), R(C-OH)-Konfiguration und, wenn n für 1 steht, die Enantiomeren mit R(C-S-), S(C-OH)-Konfiguration besonders bevorzugt. In durch das Symbol

$$-(CH=CH)_n-$$

dargestellten Vinylen- bzw. Buta-1,3-dienylenrest liegt die Doppelbindung bzw. die von dem mit dem Rest alk verbundenen C-Atom ausgehende Doppelbindung des Butadienylenrestes vorzugs-, jedoch nicht notwendigerweise in cis-Konfiguration, üblicherweise mit (Z) bezeichnet, vor, wobei die andere Doppelbindung dann vorzugs-, jedoch ebenfalls nicht notwendigerweise trans-Konfiguration, üblicherweise mit (E) bezeichnet, besitzt.

Gegebenenfalls fluoriertes Niederalkyl ist Niederalkyl oder Mono-, Di- oder Polyfluorniederalkyl.

Veräthertes bzw. verestertes Hydroxy ist beispielsweise Niederalkoxy bzw. Halogen.

Gegebenenfalls niederalkyliertes Amino ist beispielsweise Amino, Niederalkylamino oder insbesondere Diniederalkylamino.

Gegebenenfalls wie angegeben substituierte 5-gliedrige, 1 N-, O- oder S-Atom aufweisende Heteroarylreste sind beispielsweise gegebenenfalls wie angegeben substituierte Pyrryl-, wie 2-Pyrryl-, Thienyl, wie 2-Thienyl-, oder Furyl-, wie 2-Furylreste.

Gegebenenfalls verestertes oder amidiertes Carboxy ist Carboxy, verestertes Carboxy, wie Niederalkoxycarbonyl, oder amidiertes Carboxy, wie Carbamyl oder N-Mono- oder N,N-Diniederalkylcarbamoyl, oder als $R_4$ vorzugsweise im Phenylteil gegebenenfalls durch Niederalkyl, Niederalkoxy und/oder Halogen substituiertes N-(Benzolsulphonyl)carbamoyl. Im Phenylteil gegebenenfalls wie angegeben substituiertes N-(Benzolsulphonyl)-carbamoyl ist beispielsweise unsubstituiert oder, vorzugsweise in ortho-Stellung, monosubstituiert. Als gegebenenfalls veresterter oder amidierter Carboxysubstituent von $R_3$ ist Carboxy und als $R_4$ verestertes Carboxy, insbesondere Niederalkoxycarbonyl, besonders bevorzugt.

Vor und nachstehend werden unter "niederen" Resten und Verbindungen beispielsweise solche verstanden, die nicht mehr als 7 und, wenn nicht anders angegeben, vorzugsweise nicht mehr als 4 Kohlenstoffatome (C-Atome) aufweisen. Ferner gilt:

Niederalkyl ist beispielsweise $C_1$-$C_7$-Alkyl, vor allem geradkettiges $C_1$-$C_4$-Alkyl, wie Methyl, Äthyl, Propyl, Isopropyl, Butyl oder Sekundärbutyl, kann aber auch ein verzweigtes $C_1$-$C_4$-Alkyl, wie Isobutyl oder Tertiärbutyl oder Pentyl-, Hexyl- oder Heptylrest sein. Niederalkyl $R_1$, $R_5$ bzw. als Substituent von Phenyl bzw. N-(Benzolsulphonyl)carbamoyl ist vorzugsweise $C_1$-$C_4$-Alkyl, z.B. Methyl, Niederalkyl $R_2$ vorzugsweise $C_2$-$C_5$-Alkyl, z.B. Propyl.

Mono-, Di- oder Polyfluorniederalkyl weist beispielsweise bis und mit 5 Fluoratome auf und ist beispielsweise Mono-, Di- oder Trifluor-$C_1$-$C_7$-alkyl, vor allem $\omega$-Fluor- oder $\omega,\omega,\omega$-Trifluor-$C_1$-$C_4$-alkyl, wie Trifluormethyl, 2,2,2-Trifluoräthyl oder 3,3,3-Trifluorpropyl. Fluoriertes Niederalkyl $R_1$ sowie solches als Substituent von $R_3$ ist insbesondere Trifluormethyl und fluoriertes Niederalkyl $R_2$ vorzugsweise $\omega,\omega,\omega$-Trifluor-$C_2$-$C_4$-alkyl, z.B. 3,3,3-Trifluorpropyl.

Niederalkenyl $R_2$ ist beispielsweise $C_2$-$C_4$-Alkenyl, wie Vinyl, Prop-1-enyl oder insbesondere Prop-2-enyl (Allyl).

Niederalkylen ist beispielsweise geradkettiges $C_1$-$C_7$-Alkylen, im Falle von X insbesondere $C_1$-$C_3$-Alkylen, wie Methylen oder Äthylen, und im Falle von alk insbesondere $C_2$-$C_6$-Alkylen, wie Äthylen, 1,3-Propylen, 1,4-Butylen, ferner 1-5-Pentylen oder 1,6-Hexylen.

Niederalkoxy ist beispielsweise $C_1$-$C_4$-Alkoxy, wie Methoxy.

Niederalkoxycarbonyl ist beispielsweise $C_1$-$C_4$-Alkoxycarbonyl, wie Methoxy-, Äthoxy-, Propyloxy- oder Butyloxycarbonyl.

Niederalkylamino ist beispielsweise $C_1$-$C_4$-Alkylamino, wie Methyl-, Äthyl-, Propyl- oder Isopropylamino.

Diniederalkylamino ist beispielswiese Di-$C_1$-$C_4$-alkylamino, wie Dimethylamino, Diäthylamino oder N-Äthyl-N-methyl-amino.

N-Mono- oder N,N-Diniederalkylcarbamoyl ist beispielsweise N-$C_1$-$C_4$-Alkyl- oder N,N-Di-$C_1$-$C_4$-alkylcarbamyl, wie N-Methyl-, N-Äthyl-oder N,N-Dimethylcarbamyl.

Halogen ist beispielsweise Halogen der Atomnummer bis und mit 35, wie Fluor, Chlor oder Brom.

Die meisten Verbindungen der Formel I können, ihrem individuellen Charakter nach, auch in Form von Salzen vorliegen. Diejenigen davon, welche eine genügende Acidität haben, wie insbesondere die mit Carboxyl-, Tetrazolyl- oder Sulphamoylgruppen, können Salze mit Basen, wie insbesondere anorganischen Basen, vorzugsweise physiologisch verträgliche Alkalimetall-Salze, vor allem Natrium- und Kalium-Salze, bilden. In Betracht kommen aber auch Ammoniumsalze mit Ammoniak oder physiologisch verträglichen organischen Aminen, wie Mono-, Di- oder Triniederalkylaminen, z.B. Diäthylamin, Mono-, Di- oder Tri-(hydroxyalkyl)aminen, wie Tris(hydroxymethyl)methylamin, oder D-Glucosamin.

Die Verbindungen der Formel I und ihre Salze weisen vorteilhafte pharmakologische Eigenschaften, insbesondere einen ausgeprägten Leukotrienantagonismus auf.

So hemmen sie z.B. in vitro im Konzentrationsbereich von etwa 0,001-1,0 $\mu$Mol/l die durch Leukotrien-$D_4$ ($LTD_4$) induzierte Kontraktion eines glatten Muskels. Dieser sogenannte $LTD_4$-Antagonismus wird experimentell z.B. folgendermassen ermittelt: In Segmenten, die dem Ileum eines 300-400 g schweren Meerschweinchens entnommen wurden und in einem

Organbad in Tyrode-Lösung bei 38° C und unter Begasung mit einem Gemisch von 95 % Sauerstoff und 5 % Kohlenstoffdioxid bei einer Belastung von 1 g inkubiert wurden, werden mit synthetischem Leukotrien $D_4$ (als Kaliumsalz) Kontraktionen ausgelöst und isotonisch registriert. Das Ausmass der Hemmung durch die Prüfsubstanz wird nach einer Vorinkubation von 2 Minuten ermittelt und als $IC_{50}$, d.h. die Konzentration, welche die Testkontraktion um 50 % reduziert, ausgewertet. Die Verbindungen der Formel I sind auch in vivo ausgezeichnet wirksam. Sie weisen zudem den spezifischen und therapeutisch sehr bedeutsamen Vorteil einer verhältnismässig langen Wirkungsdauer auf. So konnte im in vivo Bronchokonstriktions-Standardtest am Meerschweinchen bei Aerosol-Verabreichung einer Lösung, enthaltend 0,0001 bis 1 % Gew.-% der Prüfsubstanz ein deutlicher $LTD_4$-antagonisierender Effekt nachgewiesen werden.

Ueberraschenderweise üben viele Verbindungen der Formel I auch eine ausgeprägte Hemmwirkung auf andere physiologisch wichtige Enzymsysteme aus. So wurde die Hemmung von Phospholipase $A_2$ aus menschlichen Leukocyten im getesteten Konzentrationsbereich von etwa 0,5-50 $\mu$Mol/l beobachtet. Ebenfalls wurde die Hemmung von Phospholipase C aus menschlichen Thrombocyten in getesteten Konzentrationsbereich von etwa 1-100 $\mu$Mol/l beobachtet.

Dank dieser wertvollen pharmakologischen Eigenschaften können die erfindungsgemässen Verbindungen der Formel I überall dort therapeutische Anwendung finden, wo die Wirkung der Leukotriene zu krankhaften Zuständen führt, und diese mildern oder beheben. Demzufolge können sie beispielsweise zur

Behandlung allergischer Zustände und Erkrankungen, wie insbesondere Asthma, aber auch Heufieber sowie obstruktiver Lungenkrankheiten einschliesslich zystischer Fibrose, verwendet werden. Ebenfalls sind sie, dank ihrer antiinflammatorischen Wirksamkeit, als entzündungshemmende Mittel, insbesondere als externe (topische) Hautphlogistatika für die Behandlung entzündlicher Dermatosen jeglicher Genese, wie bei leichten Hautirritationen, Kontaktdermatitis, Exanthemen und Verbrennungen, sowie als Schleimhautpphlogostatika für die Behandlung von Mukosaentzündungen, z.B. der Augen, Nase, Lippen, Mund und Genital-, bzw. Analregion, geeignet. Ferner können sie als Sonnenschutzmittel verwendet werden. Die hohe hemmende Wirksamkeit auf verschiedene Blutfaktoren weist zudem auf die Möglichkeit der therapeutischen Anwendung der Verbindungen der Formel I im Indikationsbereich Thrombose und Blutgerinnung hin.

Die Erfindung betrifft in erster Linie Verbindungen Der Formel I, worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X Niederalkylen, Oxy oder Thio darstellt, alk Niederalkylen bedeutet, $R_3$ unsubstituiertes oder durch Niederalkyl, Niederalkoxy, Halogen, Carboxy, Niederalkoxycarbonyl, Amino, N-Mono-oder N,N-Diniederalkylamino, Carbamyl, N-Mono- oder N,N-Diniederalkylcarbamyl und/oder Trifluormethyl substituiertes Pyrryl, Thienyl oder Furyl bedeutet, $R_4$ Carboxy, Niederalkoxycarbonyl, 5-Tetrazolyl, Carbamyl, N-Mono- oder N,N-Diniederalkylcarbamyl oder gegebenenfalls im Phenylteil durch Niederalkyl, Niederalkoxy, Halogen und/oder Trifluormethyl substituiertes N-(Benzolsulphonyl)carbamoyl bedeutet und $R_5$ für Wasserstoff oder Niederalkyl steht, und ihre Salze, insbesondere pharmazeutisch verwendbare Salze.

Die Erfindung betrifft vorzugsweise solche Verbindungen, worin die Gruppe X in <u>para</u> -Stellung zur $R_1$-C(=O)-Gruppe gebunden ist, d.h. Verbindungen der Formel Ia

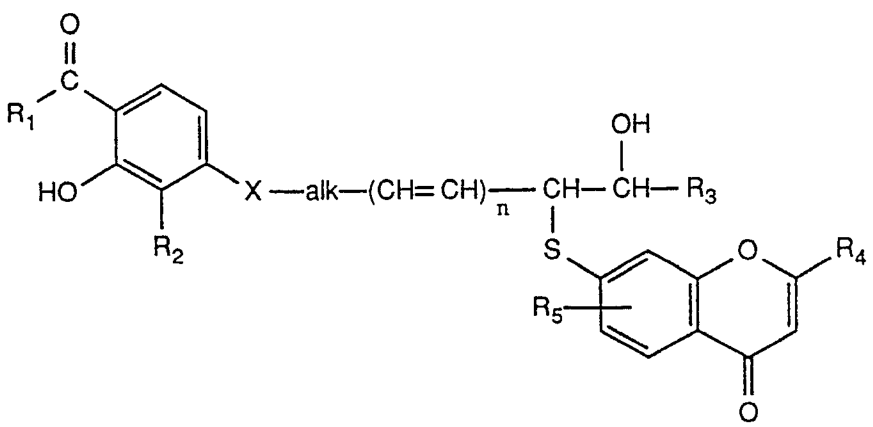

(Ia)

worin $R_1$, $R_2$, X, alk, n, $R_3$, $R_4$ und $R_5$ die angegebenen Bedeutungen haben, und ihre Salze, insbesondere pharmazeutisch verwendbare Salze.

Die Erfindung betrifft vor allem Verbindungen der Formel I bzw. Ia, worin $R_1$ $C_1$-$C_4$-Alkyl, wie Methyl, oder $\omega,\omega,\omega$-Trifluor-$C_1$-$C_4$-alkyl, wie Trifluormethyl, bedeutet, $R_2$ $C_1$-$C_4$-Alkyl, wie Propyl, $C_2$-$C_4$-Alkenyl, wie Allyl, $\omega,\omega,\omega$-Trifluor-$C_1$-$C_4$-alkyl, wie 3,3,3-Trifluorpropyl, oder in zweiter Linie Wasserstoff darstellt, X $C_1$-$C_3$-Alkylen, wie Methylen, Oxy oder Thio darstellt, alk geradkettiges $C_2$-$C_6$-Alkylen, wie Äthylen, 1,3-Propylen oder 1,4-Butylen, darstellt n für 1 oder 2 steht, $R_3$ unsubstituiertes oder durch $C_1$-$C_4$-Alkyl, wie Methyl, $C_1$-$C_4$-Alkoxy, wie Methoxy, Halogen der Atomnummer bis und mit 35, wie Chlor oder Brom, Trifluormethyl, Carboxy und/oder $C_1$-$C_4$-Alkoxycarbonyl, wie Methoxycarbonyl, substituiertes Pyrryl, wie 2-Pyrryl, Thienyl, wie 2-Thienyl, oder Furyl, wie 2-Furyl bedeutet, $R_4$ Carboxy oder N-(Benzolsulphonyl)carbamyl bedeutet, und $R_5$ für Wasserstoff steht, wobei, wenn n für 1 steht, das mit dem Schwefelatom verbundene Ketten-C-atom vorzugsweise (R)- und das mit der Hydroxygruppe verbundene Ketten-C-atom vorzugsweise (S)-Konfiguration besitzt bzw., wenn n für 2 steht, das mit dem Schwefelatom verbundene Ketten-C-Atom vorzugsweise (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die gegebenenfalls vorhandene zusätzliche Doppelbindung vorzugsweise in trans-Konfiguration vorliegt, vorzugsweise solche, in denen $R_1$ $C_1$-$C_4$-Alkyl ist und $R_2$, X, $R_3$, $R_4$ und $R_5$ die angegebenen Bedeutungen haben und ihre Salze, insbesondere pharmazeutisch verwendbaren Salze.

Die Erfindung betrifft insbesondere einerseits Verbindungen der Formel Ia, worin $R_1$ $C_1$-$C_4$-Alkyl, wie Methyl, bedeutet, $R_2$ $C_1$-$C_4$-Alkyl, wie Propyl, darstellt, X für Oxy steht, alk $C_2$-$C_6$-Alkylen, wie Äthylen, 1,3-Propylen oder 1,4-Butylen, darstellt, n für 1 oder vorzugsweise 2 steht, $R_3$ unsubstituiertes oder, insbesondere in 4-Stellung, durch $C_1$-$C_4$-Alkyl, wie Methyl, $C_1$-$C_4$-Alkoxy, wie Methoxy, Halogen der Atomnummer

bis und mit 35, wie Chlor, Trifluormethyl oder $C_1$-$C_4$-Alkoxycarbonyl, wie Methoxycarbonyl, substituiertes Pyrryl, wie 2-Pyrryl, oder insbesondere Thienyl, wie 2-Thienyl, oder Furyl, wie 2-Furyl bedeutet, $R_4$ Carboxy bedeutet, und $R_5$ Wasserstoff ist, wobei, wenn n für 1 steht, das mit dem Schwefelatom verbundene Ketten-C-atom vorzugsweise (R)- und das mit der Hydroxygruppe verbundene Ketten-C-atom vorzugsweise (S)-Konfiguration besitzt bzw., wenn n für 2 steht, das mit dem Schwefelatom verbunden Ketten-C-Atom vorzugsweise (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die gegebenenfalls vorhandene zusätzliche Doppelbindung vorzugsweise in trans-Konfiguration vorliegt, und ihre Salze, insbesondere pharmazeutisch verwendbaren Salze.

Die Erfindung betrifft insbesondere andrerseits Verbindungen der Formel Ia, worin $R_1$ $C_1$-$C_4$-Alkyl, wie Methyl, bedeutet, $R_2$ $C_1$-$C_4$-Alkyl, wie Propyl, darstellt, X für Oxy steht, alk $C_2$-$C_6$-Alkylen, wie Äthylen, 1,3-Propylen oder 1,4-Butylen, darstellt, n für 2 steht, $R_3$ unsubstituiertes oder, insbesondere in 2- und/oder 5-Stellung, durch $C_1$-$C_4$-Alkyl, wie Methyl, $C_1$-$C_4$-Alkoxy, wie Methoxy, Halogen der Atomnummer bis und mit 35, wie Chlor, und/oder Trifluormethyl mono- oder disubstituiertes Thienyl, wie 3-Thienyl, oder Furyl, wie 3-Furyl bedeutet, $R_4$ Carboxy bedeutet, und $R_5$ Wasserstoff ist, wobei das mit dem Schwefelatom verbundene Ketten-C-Atom vorzugsweise (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die gegebenenfalls vorhandene zusätzliche Doppelbindung vorzugsweise in trans-Konfiguration vorliegt, und ihre Salze, insbesondere pharmazeutisch verwendbaren Salze.

Die Erfindung betrifft in vorzugsweise Linie Verbindungen der Formel Ia, worin $R_1$ $C_1$-$C_4$-Alkyl, wie Methyl, bedeutet, $R_2$ $C_1$-$C_4$-Alkyl, wie Propyl, darstellt, X für Oxy steht, alk $C_2$-$C_5$-Alkylen, wie Äthylen, 1,3-Propylen oder 1,4-Butylen darstellt, n für 2 steht, $R_3$ unsubstituiertes oder durch $C_1$-$C_4$-Alkyl, wie Methyl, Trifluormethyl oder $C_1$-$C_4$-Alkoxycarbonyl, wie Methoxycarbonyl, insbesondere in 4-Stellung substituiertes 2- oder 3-Pyrryl oder vorzugsweise 2- oder 3-Thienyl bzw. 2- oder 3-Furyl bedeutet, $R_4$ Carboxy bedeutet, und $R_5$ Wasserstoff ist, wobei das mit dem Schwefelatom verbundene Ketten-C-Atom vorzugsweise (S)- und das di Hydroxygruppe tragende Ketten-C-Atom vorzugsweise (R)-Konfiguration besitzt und die mit dem Rest alk verbundene Doppelbindung vorzugsweise in cis-Konfiguration und die andere zusätzliche Doppelbindung vorzugsweise in trans-Konfiguration vorliegt, und ihre Salze, insbesondere pharmazeutisch verwendbaren Salze.

Die Erfindung betrifft in allererster Linie Verbindungen der Formel Ia, worin $R_1$ $C_1$-$C_4$-Alkyl, wie Methyl, bedeutet, $R_2$ $C_1$-$C_4$-Alkyl, wie Propyl, darstellt, X für Oxy steht, alk $C_2$-$C_5$-Alkylen, wie Äthylen, 1,3-Propylen oder 1,4-Butylen darstellt, n für 2 steht, $R_3$ unsubstituiertes oder, insbsondere in 2- und/oder 5-Stellung, durch Halogen der Atomnummer bis und mit 35, wie Chlor, mono- oder disubstituiertes 3-Thienyl oder 3-Furyl bedeutet, $R_4$ Carboxy bedeutet, und $R_5$ Wasserstoff ist, wobei das mit dem Schwefelatom verbundene Ketten-C-Atom vorzugsweise (S)- und das die Hydroxygruppe tragende Ketten-C-Atom vorzugsweise (R)-Konfiguration besitzt und die mit dem Rest alk verbundene Doppelbindung vorzugsweise in cis-Konfiguration und die andere zusätzliche Doppelbindung vorzugsweise in trans-Konfiguration vorliegt, und ihre Salze, insbesondere pharmazeutisch verwendbaren Salze.

Die Erfindung betrifft namentlich die in den Beispielen genannten Verbindungen der Formel I und ihre Salze, insbesondere pharmazeutisch verwendbaren Salze.

Das erfindungsgemässe Verfahren zur Herstellung von Verbindungen der Formel I und ihre Salze beruht auf an sich bekannten Methoden und ist dadurch gekennzeichnet, dass man ein Epoxid der Formel II

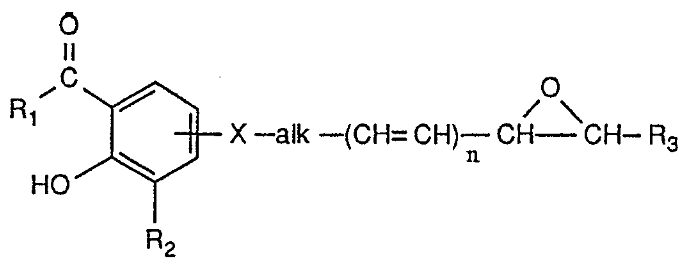

(II),

worin $R_1$, $R_2$, X, alk, n und $R_3$ obige Bedeutungen haben, mit einem Thiol der Formel III

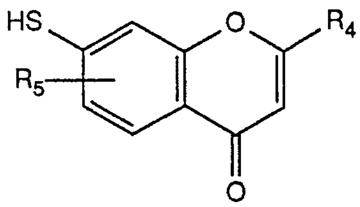

(III),

worin $R_4$ und $R_5$ obige Bedeutungen haben, oder einem Salz davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz überführt.

Die Umsetzung von Epoxiden II mit Thiolen III erfolgt unter Konfigurationsumkehr am die Bindung mit der Thiogruppe eingehenden C-Atom und unter Konfigurationserhaltung am die die Hydroxygruppe tragenden C-Atom. Um zu den bevorzugten Verbindungen mit entgegengesetzter Konfiguration an diesen beiden C-Atomen zu gelangen, geht man deshalb vorzugsweise von den entsprechenden trans-Epoxiden II aus. Dabei erhält man ausgehend von R,R-Epoxiden II Verbindungen I mit S(C-S-), R(C-OH)-Konfiguration bzw. ausgehend von S,S-Epoxiden II Verbindungen I mit R(C-S), S(C-OH)-Konfiguration. Die Umsetzung erfolgt unter an sich bekannten Bedingungen bei Temperaturen von etwa -20 °C bis etwa +50 °C, vorzugsweise bei Raumtemperatur, d.h. 18 °C bis 25 °C, und vornehmlich in einem basischen Milieu, beispielsweise in Gegenwart eines Amins, insbesondere eines tertiären aliphatischen, arylaliphatischen oder gesättigten heterocyclischen Amins, wie Trialkylamin (z.B. Triäthylamin, oder Äthyl-diisopropylamin), Dialkylbenzylamin (z.B. N,N-Dimethylbenzylamin), N,N-Dialkylanilin (z.B. N,N-Dimethylanilin) bzw. N-Methyl- oder N-Äthylpiperidin oder N,N′-Dimethylpiperazin. Ueblicherweise wird die Umsetzung in einem indifferenten organischen Lösungsmittel, wie einem Niederalkanol, z.B. Methanol oder Ethanol, durchgeführt.

In einer bevorzugten Ausführungsform geht man von Komponenten II und III aus, worin $R_4$ verestertes Carboxy oder Tetrazolyl ist und $R_3$ die angegebene Bedeutung hat, hydrolysiert $R_4$ zu Carboxy und überführt dieses gewünschtenfalls in amidiertes Carboxy.

Ausgangsstoffe für das erfindungsgemässe Verfahren sind entweder an sich bekannt oder nach bekannten Analogieverfahren in an sich bekannter Weise erhältlich.

Das als Ausgangsstoff verwendete Epoxid der oben definierten Formel II kann vornehmlich mittels derselben Verfahren hergestellt werden, welche auch bei der Synthese von Leukotrienen verwendet werden. Bei einem typischen allgemeinen Syntheseweg für Verbindungen II, worin n für 1 steht wird z.B. von einem Aldehyd der Formel

$O=CH-R_3$ (IV)

ausgegangen, worin A und $R_3$ die oben angegebenen Bedeutungen haben, wobei eine gegebenenfalls vorhandene freie Carboxylgruppe in einer als Ester geschützten Form, z.B. als Niederalkylester, vorliegt. Diese Verbindung wird mit Formylmethylentriphenylphosphoran (oder einem äquivalenten Reagens) kondensiert, wobei das entsprechende trans -3-$R_3$-Prop-2-enal der Formel

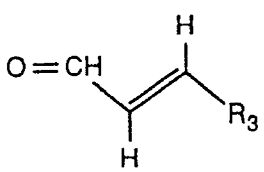

(V)

gebildet wird. Diese Verbindung wird anschliessend in an sich bekannter Weise, vorzugsweise unter schwach alkalischen Bedingungen (z.B. in Gegenwart von Alkalicarbonaten) mit wässrigem Wasserstoffperoxid epoxidiert, woraus ein trans , d.h. 2(RS),3(RS)-Epoxy-3-$R_3$-Propanal der Formel

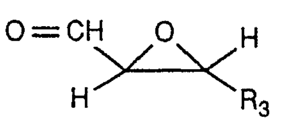

(VI)

resultiert. Der Epoxyaldehyd VI kann dann durch Kondensation mit einem Phosphoniumhalogenid

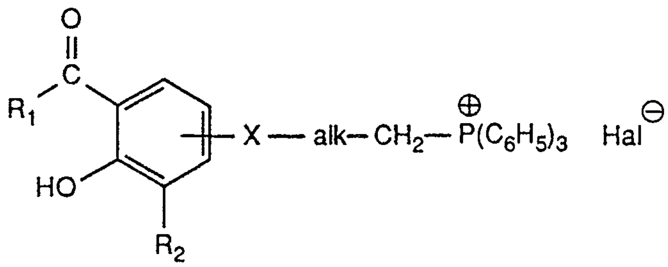

(VII)

worin $R_1$, $R_2$ und alk die angegebenen Bedeutungen haben und Hal ein Halogenatom vorzugsweise Brom, darstellt, und einer Base, z.B. Natriumamid, in Tetrahydrofuran zu dem entsprechenden Epoxid II, worin $R_4$ verestertes Carboxy bedeutet und n für 1 steht, umgesetzt werden.

Verbindungen VII werden insbesondere durch Umsetzung einer entsprechenden Verbindung der Formel

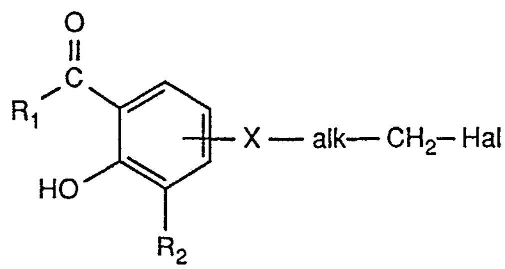

(VIII)

mit Triphenylphosphin in üblicher Weise hergestellt. Verbindungen VIII, worin X Oxy oder Thio bedeutet, werden beispielsweise erhalten, indem man entsprechende Verbindungen der Formeln

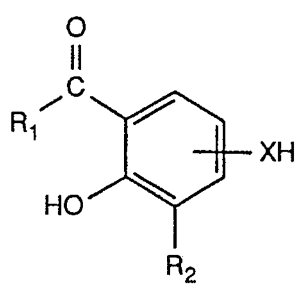

(IX) und Hal - alk-$CH_2$-Hal (X)

in üblicher Weise miteinander kondensiert.

Eine andere Methode zur Herstellung von Verbindungen II besteht darin, dass man trans -3-$R_3$-Prop-2-enol der Formel

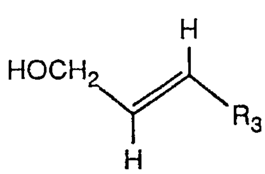

(XI),

worin $R_3$ obige Bedeutung hat, freies Carboxy als Substituent von $R_3$ aber vorzugsweise in einer Esterform geschützt ist, beispielsweise durch Behandeln mit einem N-Halogenamid, wie einem Niederalkancarbonsäure-(N-halogen)amid, z.B. mit N-Bromacetamid, oder einem aliphatischen oder aromatischen Dicarbonsäureamid, wie N-Bromsuccinimid oder N-Chlorphthalimid in das entsprechende Halohydrin der Formel

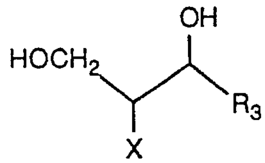

(XIa; X = Halogen)

überführt, dieses durch Basebehandlung, beispielsweise Behandlung mit Natriumhydroxid in Äthanol/Wasser, dehydrohalogniert und das erhaltene Methylolepoxid der Formel

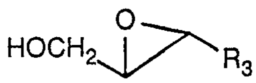

(XIb)

in üblicher Weise, beispielsweise mit Chromtrioxid in Dichlormethan, zum Epoxyaldehyd der Formel

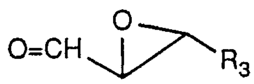

(XIc)

oxodiert.

Besonders vorteilhaft ist eine weitere Methode zur Herstellung von Zwischenprodukten der Formel II, die mit weniger Schritten zu sterisch einheitlichen Produkten führt, jedoch nicht allgemein leicht durchführbar ist. Sie beruht auf dem Prinzip, dass man das trans-Propenol XI, beispielsweise mittels Tertiärbutylhydroperoxid in Gegenwart von Titantetraisopropanolat und eines D- bzw. L-Weinsäurediniederalkylesters, epoxidiert, bei Verwendung eines D-Weinsäureesters erhält man dabei vorwiegend 2R,3R-Epoxy-3-$R_3$-propanol XIIa bzw. bei der Verwendung eines L-Weinsäureesters vorwiegend das entsprechende 2S,3S-Epoxy-3-$R_3$-propanol XIIb

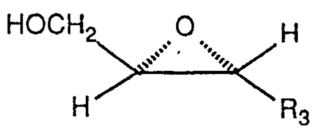

(XIIa) bzw.

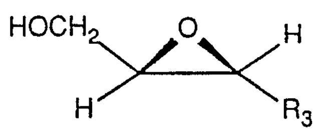

(XIIb).

Dieses wird dann, beispielsweise durch Behandeln mit Oxalylchlorid/Dimethylsulfoxid und anschliessend mit Triäthylamin, zum entsprechenden Epoxyaldehyd VI oxidiert, der dann mit dem entsprechenden Phosphoniumsalz VII in das entsprechende Epoxid II, worin $R_3$ verestertes Carboxy bedeutet und n für 1 steht, umgesetzt werden kann.

Dabei erhält man überwiegend Epoxide II, worin die Doppelbindung die bevorzugte cis-Stereotaxie besitzt. Verwendet man einen D-Weinsäureester erhält man, wie erwähnt, vorwiegend Verbindungen II, worin die Epoxygruppe R,R-Konfiguration aufweist, bzw. S,S-Enantiomere, wenn man die Umsetzung in Gegenwart von L-Weinsäureestern durchführt.

Die Herstellung von Epoxiden II, worin n für 2 steht, wird beispielsweise der Epoxyalkohol XIIa bzw. XIIb zunächst durch Behandlung mit N,N'-Dicyclohexylcarbodiimid und Dimethylsulfoxid in Gegenwart von Trifluoressigsäure und Pyridin und anschliessend mit Triphenylphosphoranylidenacetaldehyd zunächst in den entsprechende 4R,5R- bzw. 4S,5S-4,5-Epoxy-5-$R_3$-pent-2-enal der Formeln XIIIa bzw. XIIIb

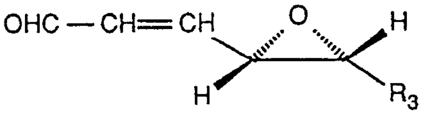

(XIIIa) bzw.

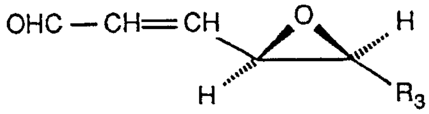

(XIIIb)

überführt, der dann mit dem Phosphoniumhalogenid VII zur entsprechenden Epoxid II, worin n für 2 steht, weiter umgesetzt wird. Dabei werden vorzugsweise solche erhalten, in denen die mit dem Rest alk verbundene Doppelbindung <u>cis</u> -Stereotaxie und die mit dem Oxiranring verbundene Doppelbindung <u>trans</u> -Stereotaxie aufweist.

Verfahrensgemäss erhältliche Verbindungen kann man gewünschtenfalls in andere Verbindungen der Formel I überführen.

Beispielsweise kann man veresterte oder amidierte Carboxygruppen zu freiem Hydroxy hydrolisieren, vorzugsweise unter basischen Bedingungen, z.B. in Gegenwart von Natronlauge, und vorzugsweise in einem mit Wasser mischbaren organischen Lösungsmittel, wie Tetrahydrofuran, Dioxan oder einem Niederalkanol, wie Methanol oder Äthanol. Ausgehend von Verbindungen I, worin $R_4$ verestertes carboxy, wie Niederalkoxycarbonyl bedeutet und $R_3$ solches als Substituenten enthält kann die Hydrolyse so gesteuert werden, dass selektiv nur $R_4$ oder sowohl $R_4$ als auch der Niederalkoxycarbonyl-substituent von $R_3$ zu Carboxy hydrolysiert werden. Verwendet man eine äquimolekulare Natronlauge und wählt milde Reaktionsbedingungen, z.B. etwa 0,5- bis 2-stündiges Rühren bei Raumtemperatur, wird praktisch nur Alkoxycarbonyl $R_4$ hydrolysiert, während unter drastischen Bedingungen, z.B. bei längeren Reaktionszeiten oder unter Erwärmen, sowohl $R_4$ als auch die Alkoxycarbonylgruppe in $R_3$ zu Carboxy hydrolysiert werden.

Umgekehrt kann man Carboxy $R_4$ sowie einen Carboxysubstituenten von $R_3$ in üblicher Weise verestern.

Freies oder verestertes Carboxy $R_4$ und solches als Substituent von $R_3$ kann ferner in üblicher Weise amidiert werden, beispielsweise durch Behandeln mit Ammoniak oder einem Mono- oder Diniederalkylamin. Beispielsweise kann man Carboxy $R_4$ in üblicher Weise, z.B. in Gegenwart eines Carbodiimidsalzes, z.B. von N-Äthyl-N′-(3-dimethylaminopropyl)-carbodiimid-hydrochlorid, und 4-Dimethylaminopyridin, mit einem gegebenenfalls substituierten Benzolsulfonamid in die entsprechende N-Benzolsulf amidoylcarbamylgruppen überführen.

Selbstverständlich kann man auch erhaltene Diastereomerengemische auf Grund unterschiedlicher physikalischer Eigenschaften der Komponenten in die individuellen Komponenten auftrennen und/oder erhaltene Enantiomerengemische nach üblichen Racemattrennungsverfahren in die individuellen Enantiomeren auftrennen.

Wenn individuelle Diastereomere erwünscht sind, so kann vorteilhaft an beliebiger Stufe ein individuelles Diastereomeres eines Ausgangsstoffes eingesetzt werden oder aus einem in Form eines Diastereomeren vorliegenden Ausgangsstoff durch stereoselektive Reaktionsbedingungen oder optisch aktive Reagentien ein Diastereomeres bevorzugt gebildet werden, oder razemische Diastereomerengemische durch physikalische Trennmethoden, gegebenenfalls unter Anwendung optisch aktiver Hilfsstoffe, in die individuelle Diastereomere aufgetrennt werden.

In stereochemischer Hinsicht wird jedoch sowohl die erfindungsgemässe Kondensation der Komponenten II und III, wie auch die Herstellung der Ausgangsstoffe vornehmlich so durchgeführt, dass man jeweils stereotaktisch einheitliche Ausgangsstoffe einsetzt, die Reaktionen nach Möglichkeit stereoselektiv, z.B. durch Einsatz von stereotaktisch einheitlichen, optisch aktiven Reagentien und/oder Hilfsstoffen, durchführt und stereotaktisch einheitliche Produkte aus Reaktionsgemischen unmittelbar nach der Reaktion isoliert. So werden z.B. bei Herstellung der ungesättigten Ausgangsstoffe gegebenenfalls gebildete cis - und trans - Isomere sofort voneinander getrennt, wozu die üblichen physikalischen Trennungsmethoden, wie insbesondere Chromatographie, geeignet sind. In der Hauptreaktion wird vornehmlich das stereomere Epoxid II mit der im Endstoff bevorzugten Stereotaxie der Doppelbindung(en) und zwar in razemischer Form (wie es bei der Variante der Epoxydierung der Verbindung V mit Wasserstoffperoxid oft gebildet wird) oder vorzugsweise als individuelles Diastereomeres mit der im Endstoff I bevorzugten Konfiguration am (C-S-)-C-Atom entgegengesetzter Konfiguration am die Bindung mit dem S-Atom eingehenden Oxiran-C-atom verwendet.

Ebenso kann man erhaltene Salze, beispielsweise durch Säurebehandlung, in die freien Säuren bzw. erhaltene freie Säuren durch Basebehandlung in Salze überführen.

Infolge der engen Beziehung zwischen den neuen Verbindungen in freier Form und in Form ihre Salze sind im voraussgegangenen und nachfolgend unter den freien Verbin dungen oder ihren Salzen sinngemäss auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder unter den Reaktionsbedingungen bildet.

Die bei den erfindungsgemässen Verfahren und ihren Vorstufen auftretenden neuen Ausgangsstoffe und Zwischenprodukte bilden ebenfalls Gegenstand der Erfindung.

Vorzugsweise werden solche Ausgangsstoffe verwendet, und die Reaktionsbedingungen so gewählt, dass man zu den vorstehend als besonders bevorzugt aufgeführten Verbindungen gelangt.

Die vorliegende Erfindung betrifft ebenfalls pharmazeutische Zusammensetzungen und Arzneimittel, welche eine der erfindungsgemässen Verbindungen der Formel I oder ein pharmazeutisch verwendbares Salz davon enthalten. Bei den erfindungsgemässen pharmazeutischen Zusammensetzungen handelt es sich

insbesondere um solche, die zur lokalen Verabreichung und vor allem zur Inhalationsverabreichung, z.B. in Form eines Aerosols, mikropulverisierten Pulvers oder einer feinversprühten Lösung, an Säuger, vor allem Menschen, bestimmt sind und den Wirkstoff allein oder zusammen mit einem pharmazeutisch anwendbaren Trägermaterial enthalten.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten). Für die Behandlung der Augen eignen sich Augentropfen, welche die aktive Verbindung in wässriger oder öliger Lösung enthalten, und Augensalben, die vorzugsweise in steriler Form hergestellt werden. Für die Behandlung der Nase sind Aerosole und Sprays (ähnlich den weiter unten beschriebenen für die Behandlung der Atemwege), grobe Puder, die durch schnelles Inhalieren durch die Nasenlöcher verabreicht werden, und vor allem Nasentropfen, welche die aktive Verbindung in wässriger oder öliger Lösung enthalten, geeigent; für die lokale Behandlung der Mundhöhle eignen sich auch Lutschbonbons, welche die aktive Verbindung in einer im allgemeinen aus Zucker und Gummiarabikum oder Tragaganth gebildeten Masse enthalten, welcher Ge schmacksstoffe beigegeben sein können, sowie Pastillen, die den Aktivstoff in einer inerten Masse, z.B. aus Gelatine und Glycerin oder Zucker und Gummiarabikum, enthalten.

Als pharmazeutische Zusammensetzungen für die Verabreichung in Form von Aerosolen oder Sprays sind geeignet z.B. Lösungen, Suspensionen oder Emulsionen des erfindungsgemässen Wirkstoffs der Formel I mit einem geeigneten pharmazeutisch annehmbaren Lösungsmittel, wie insbesondere Ethanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem einerten Gas unter erhöhtem Druck oder insbesondere mit einer leicht flüchtigen, vorzugsweise unter normalem atmosphärischem Druck unterhalb der üblichen Raumtemperatur (z.B. zwischen etwa -30 und +10 °C) siedenden Flüssigkeit, wie einem mindestens teilweise fluorierten polyhalogenierten Niederalkan, oder einem Gemisch solcher Flüssigkeiten, vermischt ist. Derartige pharmazeutische Zusammensetzungen, welche vorwiegend als Zwischenprodukte oder Vorratsgemische für die Herstellung der entsprechendenden Arzneimittel in fertiger Form verwendet werden, enthalten den Wirkstoff üblicherweise in einer Konzentration von etwa 0,1 bis etwa 10, insbesondere von etwa 0,3 bis etwa 3 Gewichts-%. - Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungsreinrichtung bzw. Ventil versehen sind. Das Ventil ist vorzugsweise als ein Dosierungsventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass entsprechende Mengen der als Vorratslösung vorliegenden pharmazeutischen Zusammensetzung und des Treibmittels separat in die Behälter abgefüllt werden und erst dort zur Vermischung kommen. Die Dosierung des zu verabreichenden Wirkstoffs der Formel I und die Häufigkeit der Verabreichung hängen ab von der jeweiligen Wirksamkeit bzw. der Länge der Wirkung jeder individueller dieser Verbindungen, von der Stärke der zu behandelnden Krankheit bzw. ihrer Symptome, sowie vom Geschlecht, Alter, Gewicht und individueller Ansprechbarkeit des zu behandelnden Säugers. Im Durchschnitt dürfte die empfohlene tägliche Dosis einer erfindungsgemässen Verbindung der Formel I bei einem 75 kg schweren Säuger (in erster Linie Menschen) im Bereich von etwa 10 bis etwa 500, vorzugsweise zwischen etwa 25 bis etwa 250 mg liegen, wobei die Verabreichung zweckmässig nach Bedarf in mehreren Dosen pro Tag erfolgen kann.

Die Erfindung betrifft auch die Anwendung der erfindungsgemässen Wirkstoffe der Formel I zur Linderung oder Behebung krankhafter Zustände und/oder Symptome des Körpers eines Säugers, insbesondere des Menschen, welche auf die Einwirkung von Leukotrienen zurückzuführen sind und insbesondere bei Asthma vorkommen. Diese Anwendung bzw. die entsprechende Heilmethode, ist durch die Behandlung des leidenden Körpers bzw. Körperteils mit einer antiallergisch wirksamen Menge einer Verbindung der Formel I allein oder in Form eines Arzneimittels, insbesondere einer zur Inhalation bestimmten pharmazeutischen Zusammensetzung, charakterisiert. Unter der Bezeichnung "eine antiallergisch wirksame Menge" ist eine solche Menge des Wirkstoffes zu verstehen, die zu einer signifikanten Inhibition der durch Leukotriene verursachten Kontraktionen ausreicht.

Die folgenden Beispiele illustrieren näher die vorliegende Erfindung, ohne sie in ihrem Umfang einzuschränken. Alle Temperaturen sind in Celsiusgraden angegeben.

Beispiel 1: 7-[(1RS,2SR)-1-Hydroxy-1-(5-chlorthiophen-2-yl)-10-(4-acetyl-3-hydroxy-2-propyl-phenoxy)-deca-3(E),5(Z)-dien-2-ylthio]-4-oxo-4H-1-benzopyran-2-carbonsäuremethylester

Die Lösung von 0,8 g (1RS,2SR)-1,2-Epoxy-1-(5-chlorthiophen-2-yl)-10-(4-acetyl-3-hydroxy-2-propyl-phenoxy)-deca-3(E),5(Z)-dien in 25 ml Methanol wird unter Argon mit 0,75 g Triäthylamin und 0,6 g 7-Mercaptochromon-2-carbonsäuremethylester 20 Stunden bei Raumtemperatur gerührt und dann eingedampft. Der Rückstand wird in Essigsäureäthylester gelöst und über Kieselgel filtriert. Das Filtrat wird mit 2n-Salzsäure und gesättigter Kochsalzlösung gewaschen, über Natriumsulfat getrocknet und eingedampft. Reinigung des Rückstandes durch Chromatographie an Kieselgel mit Essigsäureäthylester/Hexan (1:1) liefert die Titelverbindung.

Das Ausgangsmaterial kann zum Beispiel wie folgt hergestellt werden:

a) 5-Chlorthiophen-2-aldehyd wird hergestellt ausgehend von 2-Chlorthiophen analog W.J. King und F.F. Nord, J. Org. Chem. 13 , 635 (1948)

b) 3-(5-Chlorthiophen-2-yl)-propensäure wird hergestellt ausgehend von 5-Chlorthio phen-2-aldehyd gemäss a) analog W.J. King und F.F. Nord, J. Org. Chem. 14 , 405 (1949)

c) 3-(5-Chlorthiophen-2-yl)-prop-2-enol

Zu einer gerührten Suspension von 1,9 g 3-(5-Chlorthiophen-2-yl)-propensäure gemäss b) und 1,3 ml Oxalylchlorid in 40 ml Äther/Tetrahydrofuran (1:1) gibt man 0,05 ml Dimethylformamid. Die Mischung wird 2 Stunden bei Raumtemperatur gerührt, dann die klare Lösung von unlöslichem Material abdekantiert und im Vakuum vom Lösungsmittel befreit. Man erhält das rohe Säurechlorid als blassgelben Feststoff. Die Lösung diese Säurechlorids in 40 ml Äther/Tetrahydrofuran (1:1) wird zu Suspension von 250 mg Lithiumaluminiumhydrid in 30 ml Äther/Tetrahydrofuran (1:1) getropft und 2 Stunden bei Raumtemperatur gerührt. Dann wird auf 100 ml Wasser und 30 ml 1 normale Natronlauge gegossen und mit Äther (3 x 30 ml) extrahiert. Die vereinigten Extrakte werden über Natriumsulfat getrocknet, eingedampft und mit Dichlormethan/Aceton (89:1) an Kieselgel chromatographiert. Man erhält die Titelverbindung als gelbes Oel.

d) (2RS,3SR)-2,3-Epoxy-3-(5-chlorthiophen-2-yl)-propanol

Zu einer gerührten Lösung von 0,6 g 3-(5-Chlorthiophen-2-yl)-prop-2-enol gemäss c) in 50 ml Dichlormethan gibt man 0,75 g m-Chlorperbenzosäure. Die Lösung wird 5 Stunden bei Raumtemperatur gerührt, mit 10% Natriumcarbonatlösung gewaschen, über Natriumsulfat getrocknet und eingedampft. Der Rückstand wird mit Äther an Kieselgel chromatographiert und ergibt die Titelverbindung als hellgelbes Oel.

e) (2RS,3SR)-2,3-Epoxy-3-(5-chlorthiophen-2-yl)-propanal

Die Lösung von 0,6 ml Dimethylsulfoxid in 2 ml Dichlormethan wird unter Rühren bei -60° C und Stickstoffatmosphäre zur Lösung von 0,37 ml Oxalylchlorid in 10 ml Dichlormethan gegeben. Nach 5 minütigem Rühren bei -60° C gibt man innert 20 Minuten die Lösung von 0,7 g (2RS,3SR)-2,3-Epoxy-3-(5-chlorthiopheny-2-yl)-propanol gemäss d) in 10 ml Dichlormethan zu. Nach weiteren 20 Minuten bei -60° C gibt man 1,3 ml Triäthylamin zu, lässt auf Raumtemperatur erwärmen, wäscht mit Wasser und trocknet über Natriumsulfat. Nach Filtration und Eindampfen erhält man die rohe Titelverbindung als dunkles, zähes Oel.

f) (4RS,5SR)-4,5-Epoxy-5-(5-chlorthiophen-2-yl)-pent-2-enal

Die Mischung von 0,4 g rohem (2RS,3SR)-2,3-Epoxy-3-(5-chlorthiophen-2-yl)-propanal gemäss e) und 0,5 g Formyltriphenylphosphoran in 15 ml Benzol wird 4 Stunden bei Raumtemperatur gerührt. Die klare Lösung wird von Ungelöstem abdekantiert und eingedampft. Der Rückstand wird in Äther aufgenommen, von Ungelöstem abdekantiert und erneut eingedampft. Chromatographie mit Äther/Hexan (2:1) an Kieselgel liefert die Titelverbindung als blassgelben Feststoff.

g) (1RS,2SR)-1,2-Epoxy-1-(5-chlorthiophen-2-yl)-10-(4-acetyl-3-hydroxy-2-propyl-phenoxy)-deca-3(E),5(Z)-dien

Die Suspension von 5,6 g 3-(4-Acetyl-3-hydroxy-2-propyl-phenoxy)pentyltriphenylphosphoniumbromid in 80 ml Tetrahydrofuran wird mit 0,75 g Natriumhydrid und 55 mg Kaliumtertiärbutanolat unter Argon 1 Stunde bei Raumtemperatur gerührt, auf 0 - 5° C gekühlt, innerhalb von 5 Minuten mit 1,6 g (4RS,5SR)-4,5-Epoxy-5-(5-chlorthiophen-2-yl)-pent-2-enal gemäss f) in 20 ml Tetrahydrofuran versetzt und dann 2 Stunden bei Raumtemperatur gerührt. Die gebildete Suspension wird auf Phosphatpuffer (pH 7) gegossen und mit Äther extrahiert. Die vereinigten Ätherextrakte werden mit Phosphatpuffer (pH 7) gewaschen, über Natriumsulfat getrocknet und eingedampft. Der Rückstand wird in Hexan/Essigester/Triäthylamin (24:71:5) aufgenommen und über mit diesem Lösungsmittelgemisch vorgewaschenes Kieselgel filtriert. Das Filtrat wird eingedampft und ergibt die Titelverbindung als hellgelbes Oel.

Beispiel 2: 7-[(1RS,2SR)-1-Hydroxy-1-(5-chlorthiophen-2-yl)-10-(4-acetyl-3-hydroxy-2-propyl-phenoxy)-deca-3(E),5(Z)-dien-2-ylthio]-4-oxo-4H-1-benzopyran-2-carbonsäure-Natriumsalz

0,4 g des Methylesters der Titelverbindung werden unter ARgon in 10 ml Tetrahydrofuran gelöst, mit 3 ml 0,2 n-Natronlauge versetzt und 3 Stunden bei Raumtemperatur gerührt. Eindampfen und chromatographische Reinigung des Rückstandes an einer "Reversed-Phase"-Kieselgelsäule (z.B. Merck Lichroprep® RP-8) mit Methanol/Wasser (3:1) ergibt die Titelverbindung.

Beispiel 3:

Auf analoge Weise wie in den Beispielen 1 und 2 beschrieben können hergestellt werden:
7-[(1RS,2SR)-1-Hydroxy-1-(4-chlorthiophen-2-yl)-10-(4-acetyl-3-hydroxy-2-propylphenoxy)-deca-3(E),5(Z)-dien-2-ylthio]-4-oxo-4H-1-benzopyran-2-carbonsäure, ihr Methylester und ihr Natriumsalz ausgehend von 4-Chlorthiophen-2-aldehyd;
7-[(1RS,2SR)-1-Hydroxy-1-(thiophen-2-yl)-10-(4-acetyl-3-hydroxy-2-propyl-phenoxy)-deca-3(E),5(Z)-dien-2-ylthio]-4-oxo-4H-1-benzopyran-2-carbonsäure, ihr Methylester und ihr Natriumsalz ausgehend von Thiophen-2-aldehyd;
7-[(1RS,2SR)-1-Hydroxy-1-(thiophen-3-yl)-10-(4-acetyl-3-hydroxy-2-propyl-phenoxy)-deca-3(E),5(Z)-dien-2-ylthio]-4-oxo-4H-1-benzopyran-2-carbonsäure, ihr Methylester und ihr Natriumsalz ausgehend von Thiophen-3-aldehyd;
7-[(1RS,2SR)-1-Hydroxy-1-(5-fluorthiophen-2-yl)-10-(4-acetyl-3-hydroxy-2-propyl-phenoxy)-deca-3(E),5(Z)-dien-2-ylthio]-4-oxo-4H-1-benzopyran-2-carbonsäure, ihr Methylester und ihr Natriumsalz ausgehend von 5-Fluorthiophen-2-aldehyd;
7-[(1RS,2SR)-1-Hydroxy-1-(5-trifluormethylthiophen-2-yl)-10-(4-acetyl-3-hydroxy-2-propylphenoxy)-deca-3-(E),5(Z)-dien-2-ylthio]-4-oxo-4H-1-benzopyran-2-carbonsäure, ihr Methylester und ihr Natriumsalz ausgehend von 2-Trifluormethylthiophen-2-aldehyd und
7-[(1RS,2SR)-1-Hyroxy-1-(4-trifluormethylthiophen-2-yl)-10-(4-acetyl-3-hydroxy-2-propylphenoxy)-deca-3(E)-,5(Z)-dien-2-ylthio]-4-oxo-4H-1-benzopyran-2-carbonsäure, ihr Methylester und ihr Natriumsalz ausgehend von 3-Trifluormethylthiophen-2-aldehyd.

Beispiel 4: 7-[(1R,2S)-1-Hydroxy-1-(5-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäuremethylester

Die Lösung von 7,16 g (1R,2R)-1,2-Epoxy-1-(5-trifluormethylfur-2-yl)-10-(4-acetyl-3-hydroxy-2-propyl-phenoxy)-deca-3(E),5(Z)-dien in 100 ml Methanol und 7,2 ml Triäthylamin wird mit 4,02 g 7-Mercapto-4-oxo-4H-1-benzopyran-2-carbonsäuremethylester versetzt und 16 Stunden bei Raumtemperatur gerührt. Die hellbraune Suspension wird eingedampft, in 170 ml Essigsäureäthylester gelöst und über einen Kieselgelkuchen filtriert. Das Filtrat wird mit n-Salzsäure, gesättigter Kochsalzlösung, Wasser und Sole gewaschen, über Magnesiumsulfat getrocknet und eingedampft. Reinigung des Rückstandes über eine Säule (Merck, LiChroprep® Si 60) mit Hexan:Essigsäureäthylester (1:1) ergibt die Titelverbindung als hellgelben festen Schaum.

Die Ausgangsmaterialien werden wie folgt hergestellt:

a) 3-(5-Trifluormethylfur-2-yl)-2(E)-propensäuremethylester

Die Suspension von 3,15 g Natriumhydrid (80 %-ige Suspension in Weissöl) in 80 ml Tetrahydrofuran wird bei 20 - 30° innert 30 Minuten mit 20.8 ml Phosphonoessigsäuretriäthylester in 20 ml Tetrahydrofuran versetzt (exotherm, Kühlen). Nach weiteren 30 Minuten bei 20 - 30° werden 16,4 g 5-Trifluormethylfuran-2-carboxaldehyd in 25 ml Tetrahydrofuran innert 30 Minuten zugetropft und 90 Minuten bei Raumtemperatur gerührt. Das Reaktionsgemisch wird tropfenweise mit 100 ml Wasser versetzt und mit Essigsäureäthylester extrahiert. Die vereinigten organischen Phasen werden über Magnesiumsulfat getrocknet und eingedampft. Die Titelverbindung wird so als farbloses Oel erhalten.

b) 3-(5-Trifluormethylfur-2-yl)-2(E)-propenol

Die Lösung von 23,4 g 3-(5-Trifluormethylfur-2-yl)-2(E)-propensäuremethylester in 100 ml Diäthyläther wird bie 0 - 10° innert 30 Minuten mit 225 ml einer 1 molaren Diisobutylaluminiumhydrid-Lösung in Hexan versetzt. Nach weiteren 30 Minuten Rühren bei 0 - 10° wird das Reaktionsgemisch auf eine Mischung von 280 g Eis und 45 ml konz. Salzsäure gegossen und so lange gerührt, bis zwei klare Phasen entstanden sind. Trocknen der organischen Phase über Magnesiumsulfat und Eindampfen ergibt die Titelverbindung als farbloses Oel.

c) (2R,3R)-2,3-Epoxy-3-(5-Trifluormethylfur-2-yl)-propanol

Die Lösung von 18,1 g Tetraisopropylorthotitanat in 400 ml Methylenchlorid wird mit 8 g pulverisiertem Molekularsieb 4A versetzt, auf -70 bis -75° gekühlt und unter Rühren mit 15,6 g D(-)-Weinsäurediäthylester und 25,2 g 3-(5-Trifluormethylfur-2-yl)-2(E)-propenol versetzt. Nach 15-minütigem Rühren bei -70 bis -75° werden innert 20 Minuten 117 ml Tertiärbutylhydroperoxyd (3-molar in Toluol) zugetropft. Nach einer weiteren Stunde bei -65 bis -70° lässt man die Temperatur innert 2 Stunden auf 0° steigen. Die entstandene hellgelbe Lösung wird in eine Lösung von 71 g Eisen-II-sulfat-heptahydrat und 28.2 g L(+)-Weinsäure in 300 ml Wasser gegossen. Dann wird 30 Minuten bei 0 - 10° gerührt und mit Diäthyläther extrahiert. Die organischen Phasen werden über Natriumsulfat getrocknet und eingedampft. Der Rückstand wird in 450 ml Diäthyläther gelöst, bei 0 - 5° mit einer Suspension von 11,3 g Natriumhydroxid in 300 ml gesättigter Kochsalzlösung versetzt und 1 Stunde bei 0 - 5° gerüht. Die organische Phase wird abgetrennt und die wässrige Phase mit Diäthyläther extrahiert. Die vereinigten organischen Phasen werden getrocknet und eingedampft. Der Rückstand wird über eine Kieselgelsäule (Merck LiChroprep® Si 60) mit Hexan/Essigsäureäthylester (1:1) gereinigt. Die Titelverbindung wird so als farbloses Oel erhalten.

d) (4R,5R)-4,5-Epoxy-5-(5-Trifluormethylfur-2-yl)-pent-2(E)-enal

Die Lösung von 65,3 g (2R,3R)-2,3-Epoxy-3-(5-Trifluormethylfur-2-yl)-propanol in 700 ml Dimethyslfulfoxyd und 96 ml Triäthylamin wird bei 20 - 22° innert 30 Minuten mit 110 g Schwefeltrioxyd-Pyridin-Komplex gelöst in 350 ml Dimethylsulfoxyd versetzt (exotherm, Kühlen). Nach weiteren 30 Minuten Rühren werden innert 1 Stunde 86 g Formylmethylentriphenylphosphoran portionenweise zugegeben und 18 Stunden bei Raumtemperatur gerüht. Das Reaktionsgemisch wird nach der Zugabe von 2L Essigsäureäthylester mit Phosphatpuffer (pH 7) gewaschen. Die organische Phase wird über Natriumsulfat getrocknet und eingedampft. Der Rückstand wird in 400 ml Hexan/Essigsäureäthylester (4:1) suspendiert und nach 15 minütigem Rühren abgesaugt. Der Niederschlag wird mit Hexan/Essigsäureäthylester (4:1) gewaschen und das Filtat eingedampft. Der Rückstand wird über eine Säule (Merck LiChroprep® Kieselgel 60) mit Hexan/Essigsäureäthylester (3:1) gereinigt. Die Titelverbindung wird so als hellgelbes Oel erhalten.

e) (1R,2R)-1,2-Epoxy-1-(5-trifluormethylfur-2-yl)-10-(4-acetyl-3-hydroxy-2-propyl-phenoxy)-deca-3(E),5(Z)-dien

Die Suspension von 31.9 g 3-(4-acetyl-3-hydroxy-2-propyl-phenoxy)-pentyltriphenylphosphoniumbromid in 120 ml Tetrahydrofuran wird mit 8,63 g (4R,5R)-4,5-Epoxy-5-(5-Trifluormethylfur-2-yl)-pent-2(E)-enal in 20 ml Tetrahydrofuran und anschliessend mit 4,16 g Natriumamid-Pulver in kleinen Portionen versetzt. Das Reaktionsgemisch wird 2 Stunden bei 20 - 25° gerührt und abgesaugt. Der Niederschlag wird mit Tetrahydrofuran gewaschen. Das Filtrat wird mit Phosphatpuffer (pH 7) versetzt und mit Diäthyläther

extrahiert. Die vereinigten organischen Phasen werden über Natriumsulfat getrocknet und eingedampft. Der Rückstand wird in 250 ml Hexan/Essigsäureäthylester + 1 % Triäthylamin aufgenommen und über einen Kieselgelkuchen, der mit dem gleichen Lösungsmittelgemisch vorgewaschen wurde, filtriert. Das Filtrat wird eingedampft und der Rückstand über eine Säule (Merck LiChroprep® Si 60) mit Hexan/Essigsäureäthylester (7:3 + 1 % Triäthylamin) gereinigt. Die Titelverbindung wird als hellgelbes Oel erhalten.

Beispiel 5: 7-[(1R,2S)-1-Hydroxy-1-(5-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure-Natriumsalz

0.71 g des Methylesters aus Beispiel 4 werden unter Argon in 20 ml Tetrahydrofuran gelöst, mit 5 ml 0.2 N NaOH versetzt und 1 Stunde bei Raumtemperatur gerührt. Eindampfen und Reinigung des Rückstandes über eine Säule (MERCK LiChroprep RP-8) mit MeOH:$H_2O$ = 3:1 ergibt die Titelverbindung. Smp.: 204 - 207°.

Beispiel 6:

In analoger Weise wie in den Beispielen 1 - 5 beschrieben können hergestellt werden:
7-[(1R,2S)-1-Hydroxy-1-(5-trifluormethylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-methylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-methylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-chlorfur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-chlorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-fluorfur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-fluorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-methoxycarbonylfur-2-yl)-10-(4-acetyl-3-hydroxy-2-propyl-phenoxy)-deca-3(E),5-(Z)-dien-2-ylthio]-4-oxo-4H-1-benzopyran-2-carbonsäure und ihr Natriumsalz;
7-[(1R,2S)-1-Hydroxy-1-(5-methoxycarbonylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-carboxyfur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Dinatriumsalz;
7-[(1R,2S)-1-Hydroxy-1-(5-carboxyfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Dinatriumsalz;
7-[(1R,2S)-1-Hydroxy-1-(4-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(4-trifluormethylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(4-methylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(4-methylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(4-chlorfur-2-yl)-10-(4-acetyl-3-hydroxy-2-propyl-phenoxy-deca-3(E),5(Z)-dien-2-ylthio]-4-oxo-4H-1-benzopyran-2-carbonsäure und ihr Natriumsalz;
7-[(1R,2S)-1-Hydroxy-1-(4-chlorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(4-fluorfur-2-yl)-10-(4-acetyl-3-hydroxy-2-propylphenoxy)-deca-3(E).5(Z)-dien-2-ylthio]-4-oxo-4H-1-benzopyran-2-carbonsäure und ihr Natriumsalz;
7-[(1R,2S)-1-Hydroxy-1-(4-fluorfur-2-yl)-8-(4-acetyl-3-hydroxy-2-propyl-phenoxy-octa-3(E),5(Z)-dien-2-ylthio]-4-oxo-4H-1-benzopyran-2-carbonsäure und ihr Natriumsalz;

7-[(1R,2S)-1-Hydroxy-1-(4-methoxycarbonylfur-2-yl)-10-(4-acetyl-3-hydroxy-2-propyl-phenoxy)-deca-3(E),5-(Z)-dien-2-ylthio]-4-oxo-4H-1-benzopyran-2-carbonsäure und ihr Natriumsalz;
7-[(1R,2S)-1-Hydroxy-1-(4-methoxycarbonylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(4-carboxy-fur-2-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Dinatriumsalz;
7-[(1R,2S)-1-Hydroxy-1-(4-carboxyfur-2-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Dinatriumsalz;
7-[(1R,2S)-1-Hydroxy-1-(5-trifluormethylfur-3-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-trifluormethylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-methylfur-3-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-methylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-chlorfur-3-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-chlorfur-3-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-fluorfur-3-yl)-10-(4-acetyl-3-hydroxy-2-propyl-phenoxy)-deca-3(E),5(Z)-dien-2-ylthio]-4-oxo-4H-1-benzopyran-2-carbonsäure und ihr Natriumsalz;
7-[(1R,2S)-1-Hydroxy-1-(5-fluorfur-3-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-methoxycarbonylfur-3-yl)-10-(4-acetyl-3-hydroxy-2-propyl-phenoxy)-deca-3(E),5-(Z)-dien-2-ylthio]-4-oxo-4H-1-benzopyran-2-carbonsäure und ihr Natriumsalz;
7-[(1R,2S)-1-Hydroxy-1-(5-methoxycarbonylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Natriumsalz;
7-[(1R,2S)-1-Hydroxy-1-(5-carboxyfur-3-yl)-10-(4-acetyl-3-hydroxy-2-propylphenoxy)-deca-3(E),5(Z)-dien-2-ylthio]-4-oxo-4H-1-benzopyran-2-carbonsäure und ihr Dinatriumsalz sowie
7-[(1R,2S)-1-Hydroxy-1-(5-carboxyfur-3-yl)-8-(4-acetyl-3-hydroxy-2-propyl-phenoxy)-octa-3(E),5(Z)-dien-2-ylthio]-4-oxo-4H-1-benzopyran-2-carbonsäure und ihr Dinatriumsalz.

Beispiel 7: 7-[(1RS,2SR)-1-Hydroxy-1-(thiophen-3-yl)-8-(4-acetyl-3-hydroxy-2-propyl-phenoxy)-octa-3(E),5-(Z)-dien-2-ylthio]-4-oxo-4H-1-benzopyran-2-carbonsäuremethylester

Die Lösung von 0,4 g (1RS,2SR)-1,2-Epoxy-1-(thiophen-3-yl)-8-(4-acetyl-3-hydroxy-2-propylphenoxy)-octa-3(E),5(Z)-dien in 10 ml Methanol wird unter Argon mit 0,37 g Triäthylamin und 0,3 g -7-Mercapto-chromon-2-carbonsäure-methylester 20 Stunden bei Raumtemperatur gerührt und dann eingedampft. Der Rückstand wird in Essigsäureäthylester gelöst und über Kieselgel filtriert. Das Filtrat wird mit 2n-Salzsäure und gesättigter Kochsalzlösung gewaschen, über Natriumsulfat getrocknet und eingedampft. Reinigung des Rückstandes durch Chromatographie an Kieselgel mit Essigsäureäthylester/Hexan (1:1) liefert die Titelverbindung.

Das Ausgangsmaterial kann zum Beispiel wie folgt hergestellt werden:

a) 3-(Thiophen-3-yl)-prop-2-enol

Zu einer gerührten Suspension von 10,9 g 3-(Thiophen-3-yl)-propensäure und 9,3 ml Oxalylchlorid in 80 ml Ether/Tetrahydrofuran (1:1) gibt man 0,1 ml Dimethylformamid. Die Mischung wird 2 Stunden bei Raumtemperatur gerührt, dann die klare Lösung von unlöslichem Material abdekantiert und im Vakuum vom Lösungsmittel befreit. Man erhält das Säurechlorid als blassgelben Feststoff. Die Lösung dieses Säurechlorids in 80 ml Ether/Tetrahydrofuran (1:1) wird zur Suspension von 1,9 g Lithiumaluminiumhydrid in 100 ml Ether/Tetrahydrofuran (1:1) getropft und 2 Stunden bei Raumtemperatur gerührt. Dann wird auf 800 ml Wasser und 200 ml 1 normale Natronlauge gegossen und mit Ether (3 x 100 ml) extrahiert. Die vereinigten Extrakte werden über Natriumsulfat getrocknet, eingedampft und mit Dichlormethan/Aceton (9:1) am Kieselgel chromatographiert. Man erhält die Titelverbindung als hellgelbe Kristalle, Smp. = 58-60 °C.

b) (2RS,3SR)-2-Brom-3-(thiophen-3-yl)-propan-1,3-diol

Zu einer bei 0° C gerührten Lösung von 9,5 g 3-(Thiophen-3-yl)-prop-2-enol in 300 ml Aceton gibt man 11,0 g N-Bromacetamid in 100 ml Wasser. Die Reaktionslösung wird 14 Stunden bei Raumtemperatur gerührt, dass Aceton im Vakuum abgedämpft und der wässrig-ölige Rückstand mit 300 ml Ether extrahiert. Nach Trocknen über Natriumsulfat und Abdampfen des Lösungsmittels wird der Rückstand mit Dichlormethan/Aceton (4:1) an Kieselgel chromatographiert. Man erhält die Titelverbindung als gelbes Oel.

c) (2RS,3SR)-2,3 Epoxy-3-(thiophen-3-yl)-propanol

Zur Lösung von 2,4 g (2RS,3SR)-2-Brom-3-(thiophen-3-yl)-propan-1,3-diol in 25 ml Ethanol gibt man 0,8 g Natriumhydroxid in 15 ml Wasser. Man rührt 40 Minuten bei Raumtemperatur, dampft das Ethanol im Vakuum ab und verdünnt den Rückstand mit 400 ml Wasser. Man extrahiert mit Dichlormethan (3 x 100 ml), trocknet die Extrakte über Natriumsulfat und dampft das Lösungsmittel ab. Der Rückstand wird mit Dichlormethan/Aceton (9:1) an Kieselgel chromatographiert. Man erhält die Titelverbindung als hellgelbe Kristalle, Smp. = 63-65° C.

d) (2RS,3SR)-2,3 Epoxy-3-(thiophen-3-yl)-propanol

Zur Lösung von 26,5 g Chromtrioxid in 42 ml Pyridin und 660 ml Dichlormethan gibt man 6,9 g (2RS,3SR)-2,3 Epoxy-3-(thiophen-3-yl)-propanol gelöst in 35 ml Dichlormethan. Man rührt die Reaktionsmischung 15 Minuten bei Raumtemperatur, dekantiert von Ungelöstem ab und wäscht die Lösung nacheinander mit je 100 ml 5 % Natronlauge, 5 % Salzsäure, 5 % Natriumhydrogencarbonatlösung und gesättigter Kochsalzlösung. Nach Trocknen über Natriumsulfat und Abdampfen des Lösungsmittels im Vakuum erhält man die Titelverbindung als gelbes Oel.

e) (4RS,5SR)-4,5-Epoxy-5-(thiophen-3-yl)-pent-2-enal

Die Lösung von 5,5 g (2RS,3SR)-2,3-Epoxy-3-(thiophen-3-yl)-propanol in 300 ml Dichlormethan wird mit 11,0 g Formyltriphenylphosphoran versetzt und 10 Stunden bei Raumtemperatur gerührt. Das Reaktionsgemisch wird mit Phosphatpuffer pH 7 gewaschen, über Natriumsulfat getrocknet und im Vakuum vom Lösungsmittel befreit. Der Rückstand mit Hexan/Ethylacetat (4:1) an Kieselgel chromatographiert. Man erhält die Titelverbindung als gelbes Oel.

f) (1RS,2SR)-1,2-Epoxy-1-(thiophen-3-yl)-8-(4-acetyl-3-hydroxy-2-propylphenoxy)-octa-3(E),5(Z)-dien

Die Suspension von 5,0 g 3-(4-Acetyl-3-hydroxy-2-propylphenoxy)propyltriphenylphosphoniumbromid in 80 ml Tetrahydrofuran wird mit 0,73 g Natriumamid und 52 mg Kaliumtertiärbutanolat unter Argon 1 Stunde bei Raumtemperatur gerührt, auf -100° C abgekühlt, innerhalb von 5 Minuten mit 1,5 g (4RS,5SR)-4,5-Epoxy-5-(thiophen-3-yl)-pent-2-enal in 20 ml Tetrahydrofuran versetzt und dann 2 Stunden bei Raumtemperatur gerührt. Die entstandene Suspension wird auf Phosphatpuffer (pH 7) gegossen und mit Äther extrahiert. Die vereinigten Ätherextrakte werden mit Phosphatpuffer pH 7 gewaschen, über Natriumsulfat getrocknet und eingedampft. Der Rückstand wird in Hexan/Essigester/Triäthylamin (24:71:5) aufgenommen und über mit diesem Lösungsmittelgemisch vorgewaschenes Kieselgel filtriert. Das Filtrat wird eingedampft und ergibt die Titelverbindung als hellgelbes Oel.

Beispiel 8: (1RS,2SR)-1-Hydroxy-1-(thiophen-3-yl)-8-(4-acetyl-3-hydroxy-2-propyl-phenoxy)-octa-3 (E),5(Z)-dien-2-yl-7-thio-4-oxo-4H-1-benzopyran-2-carbonsäure-Natriumsalz

0,4 g des Methylesters der Titelverbindung gemäss Beispiel 7 werden unter Argon in 10 ml Tetrahydrofuran gelöst, mit 3 ml 0,2 n-Natronlauge versetzt und 3 Stunden bei Raumtemperatur gerührt. Eindampfen und chromatographische Reinigung des Rückstandes an einer "Reversed-Phase"-Kieselgelsäule (z.B. Merck Lichroprep® RP-8) mit Methanol/Wasser (3:1) ergibt die Titelverbindung als hellgelben Feststoff vom

Smp. 200-203°.

Beispiel 9: (1RS,2SR)-1-Hydroxy-1-(thiophen-3-yl)-8-(4-acetyl-3-hydroxy-2-propyl-phenoxy)-octa-3(E),5(Z)-dien-2-yl-7-thio-4-oxo-4H-1-benzopyran-2-carbonsäure

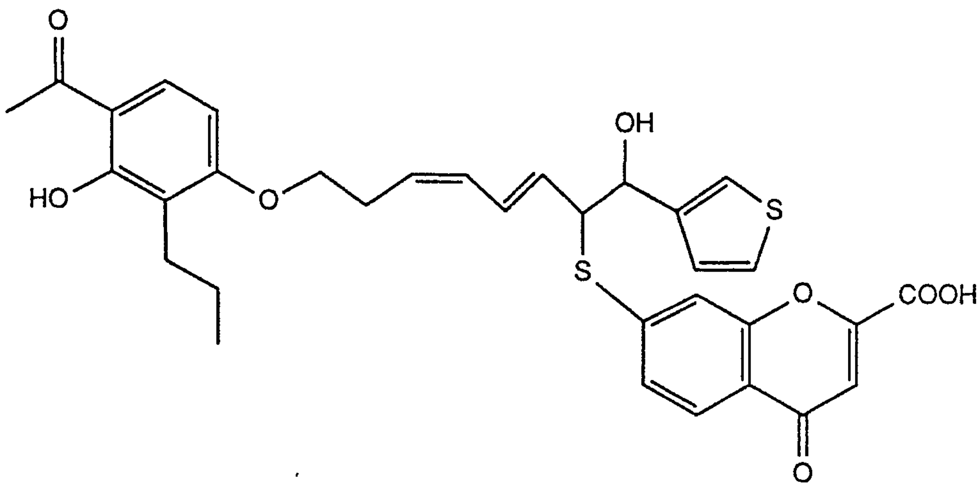

0,5 g des Methylesters der Titelverbindung (Beispiel 7) werden unter Argon in 10 ml Tetrahydrofuran gelöst, mit 4 ml 0,2 n-Natronlauge versetzt und 3 Stunden bei Raumtemperatur gerührt. Das Reaktionsgemisch wird mit 50 ml Wasser verdünnt, mit 5 ml 0,2n-Salzsäure sauer gestellt und mit Dichlormethan extrahiert. Nach Trocknen über Natriumsulfat und Abdampfen des Lösungsmittels, wird der Rückstand mit Chloroform/Methanol (4:1) an Kieselgel chromatographiert. Man erhält die Titelverbindung als hellgelbes Harz.

Beispiel 10:

In analoger Weise wie in den Beispielen 7 bis 9 beschrieben können ferner hergestellt werden:
7-[(1RS,2SR)-Hydroxy-1-(fur-3-yl)-10-(4-acetyl-3-hydroxy-2-propylphenoxy)-deca-3(E),5(Z)-dien-2-ylthio]-4-oxo-4H-1-benzopyran-2-carbonsäure und ihr Natriumsalz;
7-[(1RS,2SR)-Hydroxy-1-(fur-3-yl)-9-(4-acetyl-3-hydroxy-2-propylphenoxy)-nona-3(E),5(Z)-dien-2-ylthio]-4-oxo-4H-1-benzopyran-2-carbonsäure und ihr Natriumsalz;
7-[(1RS,2SR)-Hydroxy-1-(fur-3-yl)-8-(4-acetyl-3-hydroxy-2-propylphenoxy)-octa-3(E),5(Z)-dien-2-ylthio]-4-oxo-4H-1-benzopyran-2-carbonsäure und ihr Natriumsalz;
7-[(1RS,2SR)-Hydroxy-1-(2,5-dichlorthiophen-3-yl)-10-(4-acetyl-3-hydroxy-2-propylphenoxy)-deca-3(E),5(Z)-dien-2-ylthio]-4-oxo-4H-1-benzopyran-2-carbonsäure und ihr Natriumsalz.

Beispiel 11:

Eine treibmittelhaltige, feststoffaerosolbildende Inhalationssuspension enthaltend 0,1 Gew.-% Wirkstoff, z.B. 7-[(1RS,2SR)-1-Hydroxy-(5-chlorthiophen-2-yl)-10-(4-acetyl-3-hydroxy-2-propyl-phenoxy)-deca-3(E),5-(Z)-dien-2-ylthio]-4-oxo-4H-1-benzopyran-2-carbonsäure-Natriumsalz, kann z.B. folgendermassen hergestellt werden:

| Zusammensetzung: | Gew.-% |
|---|---|
| Wirkstoff, mikronisiert<br>Sorbitantrioleat | 0,1<br>0,5 |
| Treibmittel A | |
| (Trichlortrifluorethan) | 4,4 |
| Treibmittel B | |
| (Dichlordifluormethan und<br>1,2-Dichlortetrafluorethan) | 15,0<br>80,0 |

Herstellung:

Der Wirkstoff wird unter Feuchtigkeitsausschluss mit Hilfe eines üblichen Homogenisators unter Zusatz des Sorbitantrioleats im Trichlortrifluorethan suspendiert, die Suspension in einen mit Dosierventil versehenen Aerosolbehälter abgefüllt; dieser wird verschlossen und unter Druck mit dem Treibmittel B aufgefüllt.

Beispiel 12:

Eine zur Inhalation geeignete, etwa 2%ige wässrige Lösung eines Wirkstoffes in Form dessen Natrium- oder Kalium-salzes, z.B. von7-[1RS,2SR)-1-Hydroxy-(5-chlorthiophen-2-yl)-10-(4-acetyl-3-hydroxy-2-propylphenoxy)-deca-3(E),5(Z)-dien-2-ylthio]-4-oxo-4H-1-benzopyran-2-carbonsäurenatriumsalz, kann z.B. folgendermassen hergestellt werden:

| Zusammensetzung | |
|---|---|
| Wirkstoff (K- oder Na-Salz)<br>Aethylendiamintetraessigsäure-Dinatriumsalz<br>Benzalkoniumchlorid<br>Wasser, frisch destilliert ad | 2000 mg<br>10 mg<br>10 mg<br>100 ml |

Herstellung:

Der Wirkstoff wird in etwa 60 ml frisch destilliertem Wasser gelöst und der Stabilisator (Äthylendiamintetraessigsäure-Dinatriumsalz) und das Konservierungsmittel (Benzalkoniumchlorid) hinzugegeben. Nach vollständiger Auflösung aller Komponenten wird die erhaltene Lösung auf 100 ml aufgefüllt, in Druckfläschchen abgefüllt und diese gasdicht verschlossen. Das Treibmittel wird nach Bedarf gasförmig unter Druck oder in flüssiger Form zugegeben.

## Ansprüche

1. Neue substituierte Alkanophenone der allgemeinen Formel

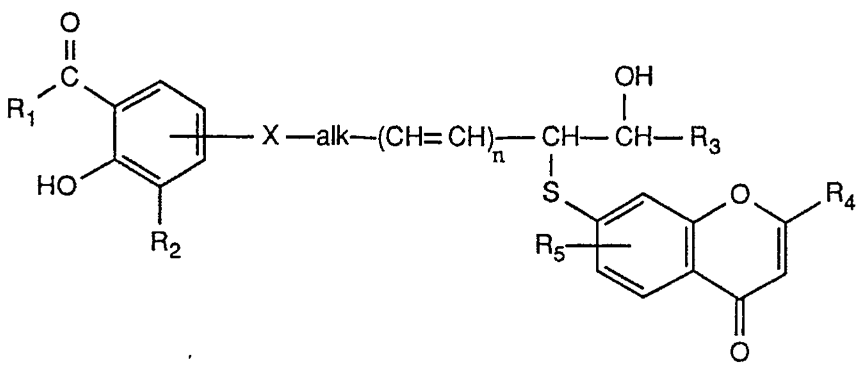

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ einen unsubstituierten oder durch gegebenenfalls fluoriertes Niederalkyl, veräthertes oder verestertes Hydroxy, Halogen, gegebenenfalls niederalkyliertes Amino und/oder gegebenenfalls verestertes oder amidiertes Carboxy substituierten 5-gliedrigen, als Heteroatom 1 N-, O- oder S-Atom aufweisenden Heteroarylrest bedeutet, $R_4$ gegebenenfalls verestertes oder amidiertes Carboxy oder 5-Tetrazolyl darstellt und $R_5$ für Wasserstoff oder Niederalkyl steht, und ihre Salze.

2. Verbindungen gemäss Anspruch 1, der Formel I, worin $R_1$ Niederalkyl oder Mono-, Di-oder Polyfluorniederalkyl bedeutet, $R_2$ Wasserstoff, Niederalkyl, Niederalkenyl oder Mono-, Di- oder Polyfluorniederalkyl darstellt, X Niederalkylen, Oxy oder Thio darstellt alk Niederalkylen bedeutet, $R_3$ unsubstituiertes oder durch Niederalkyl, Niederalkoxy, Halogen, Carboxy, Niederalkoxycarbonyl, Amino, N-Mono- oder N,N-Diniederalkylamino, Carbamyl, N-Mono-oder N,N-Diniederalkylcarbamyl und/oder Trifluormethyl substituiertes Pyrryl, Thienyl oder Furyl bedeutet, $R_4$ Carboxy, Niederalkoxycarbonyl, 5-Tetrazolyl, Carbamyl, N-Mono- oder N,N-Diniederalkylcarbamyl oder gegebenenfalls im Phenylteil durch Niederalkyl, Niederalkoxy, Halogen und/oder Trifluormethyl substituiertes N-(Benzolsulphonyl)carbamoyl bedeutet und $R_5$ für Wasserstoff oder Niederalkyl steht, und ihre Salze.

3. Verbindungen gemäss Anspruch 1, der Formel I, worin $R_1$ $C_1$-$C_4$-Alkyl oder ω,ω,ω-Trifluor-$C_1$-$C_4$-alkyl bedeutet, $R_2$ Wasserstoff, $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt n für 1 oder 2 steht, $R_3$ unsubstituiertes oder durch $C_1$-$C_4$-Alkyl, $C_1$-$C_4$-Alkoxy, Halogen der Atomnummer bis und mit 35, Trifluormethyl, Carboxy und/oder $C_1$-$C_4$-Alkoxycarbonyl, substituiertes Pyrryl, Thienyl oder Furyl bedeutet, $R_4$ Carboxy oder N-(Benzolsulphonyl)carbamyl bedeutet, und $R_5$ für Wasserstoff steht, und ihre Salze.

4. Verbindungen gemäss Anspruch 1 der Formel

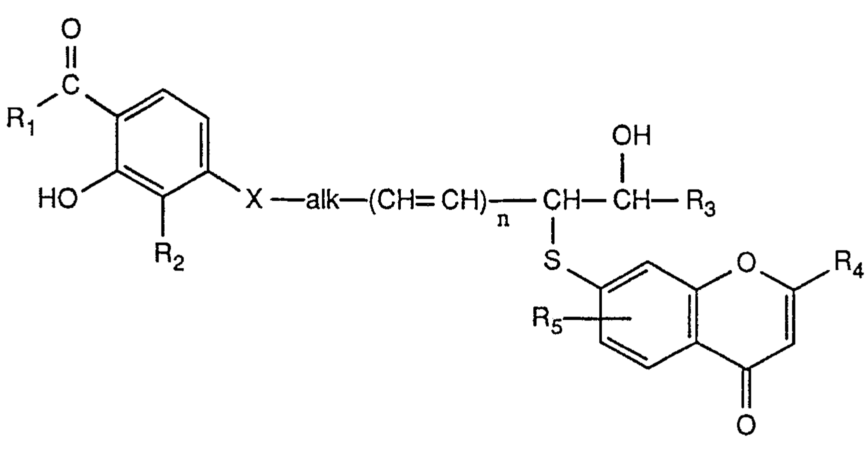

(Ia),

worin $R_1$ $C_1$-$C_4$-Alkyl, bedeutet, $R_2$ $C_1$-$C_4$-Alkyl darstellt, X für Oxy steht, alk $C_2$-$C_6$-Alkylen darstellt, n für 1 oder 2 steht, $R_3$ unsubstituiertes oder durch $C_1$-$C_4$-Alkyl, $C_1$-$C_4$-Alkoxy, Halogen der Atomnummer bis und mit 35, Trifluormethyl oder $C_1$-$C_4$-Alkoxycarbonyl substituiertes Pyrryl, Thienyl oder Furyl bedeutet, $R_4$ Carboxy bedeutet, und $R_5$ Wasserstoff ist, und ihre Salze.

5. Verbindungen gemäss Anspruch 4, der Formel Ia, worin $R_1$ $C_1$-$C_4$-Alkyl bedeutet, $R_2$ $C_1$-$C_4$-Alkyl darstellt, X für Oxy steht, alk $C_2$-$C_6$-Alkylen darstellt, n für 2 steht, $R_3$ unsubstituiertes oder durch $C_1$-$C_4$-

Alkyl, $C_1$-$C_4$-Alkoxy, Halogen der Atomnummer bis und mit 35 und/oder Trifluormethyl mono- oder disubstituiertes Thienyl oder Furyl bedeutet, $R_4$ Carboxy bedeutet und $R_5$ Wasserstoff ist, und ihre Salze.

6. Verbindungen gemäss Anspruch 4, der Formel Ia, worin $R_1$ $C_1$-$C_4$-Alkyl bedeutet, $R_2$ $C_1$-$C_4$-Alkyl darstellt, X für Oxy steht, alk $C_2$-$C_5$-Alkylen darstellt, n für 2 steht, $R_3$ unsubstituiertes oder durch Halogen der Atomnummer bis und mit 35 mono- oder disubstituiertes 3-Thienyl oder 3-Furyl bedeutet, $R_4$ Carboxy bedeutet und $R_5$ Wasserstoff ist, und ihre Salze, insbesondere pharmazeutisch verwendbaren Salze.

7. Eine Verbindung gemäss einem der Ansprüche 1 bis 6, worin die mit dem Rest alk verbundene Doppelbindung in (Z)-, d.h. cis-Konfiguration und die gegebenenfalls vorhandene zusätzliche Doppelbindung in (E)-, d.h. trans-Konfiguration vorliegt.

8. Eine Verbindung gemäss einem der Ansprüche 1 bis 7, worin das mit dem Schwefelatom verbundene Ketten-C-Atom (S)- und das die Hydroxygruppe tragende Ketten-C-Atom (R)-Konfiguration besitzt.

9. 7-[(1RS,2SR)-1-Hydroxy-(thiophen-3-yl)-10-(4-acetyl-3-hydroxy-2-propyl-phenoxy)-deca-3(E),5(Z)-dien-2-ylthio]-4-oxo-4H-1-benzopyran-2-carbonsäuremethylester.

10. 7-[(1RS,2SR)-1-Hydroxy-(thiophen-3-yl)-10-(4-acetyl-3-hydroxy-2-propyl-phenoxy)-deca-3(E),5(Z)-dien-2-ylthio]-4-oxo-4H-1-benzopyran-2-carbonsäure oder ein Salz davon.

11. 7-[(1RS,2SR)-1-Hydroxy-1-(thiophen-3-yl)-8-(4-acetyl-3-hydroxy-2-propylphenoxy)-octa-3(E),5(Z)-dien-2-ylthio]-4-oxo-4H-1-benzopyran-2-carbonsäuremethylester

12. 7-[(1RS,2SR)-1-Hydroxy-1-(thiophen-3-yl)-8-(4-acetyl-3-hydroxy-2-propylphenoxy)-octa-3(E),5(Z)-dien-2-ylthio]-4-oxo-4H-1-benzopyran-2-carbonsäure oder ein Salz davon.

13. 7-[(1RS,2SR)-Hydroxy-1-(fur-3-yl)-10-(4-acetyl-3-hydroxy-2-propylphenoxy)-deca-3(E),5(Z)-dien-2-ylthio]-4-oxo-4H-1-benzopyran-2-carbonsäure oder ein Salz davon.

14. 7-[(1RS,2SR)-Hydroxy-1-(fur-3-yl)-9-(4-acetyl-3-hydroxy-2-propylphenoxy)-nona-3(E),5(Z)-dien-2-ylthio]-4-oxo-4H-1-benzopyran-2-carbonsäure oder ein Salz davon.

15. 7-[(1RS,2SR)-Hydroxy-1-(fur-3-yl)-8-(4-acetyl-3-hydroxy-2-propylphenoxy)-octa-3(E),5(Z)-dien-2-ylthio]-4-oxo-4H-1-benzopyran-2-carbonsäure oder ein Salz davon.

16. 7-[(1RS,2SR)-Hydroxy-1-(2,5-dichlorthiophen-3-yl)-10-(4-acetyl-3-hydroxy-2-propyl phenoxy)-deca-3(E)-,5(Z)-dien-2-ylthio]-4-oxo-4H-1-benzopyran-2-carbonsäuremethylester.

17. 7-[(1RS,2SR)-Hydroxy-1-(2,5-dichlorthiophen-3-yl)-10-(4-acetyl-3-hydroxy-2-propyl-phenoxy)-deca-3(E)-,5(Z)-dien-2-ylthio]-4-oxo-4H-1-benzopyran-2-carbonsäure oder ein Salz davon.

18. Eine Verbindung ausgewählt aus:

7-[(1R,2S)-1-Hydroxy-1-(5-chlorthiophen-2-yl)-10-(4-acetyl-3-hydroxy-2-propyl-phenoxy)-deca-3-3(E),5(Z)-dien-2-ylthio]-4-oxo-4H-1-benzopyran-2-carbonsäure,

7-[(1R,2S)-1-Hydroxy-1-(5-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1RS,2SR)-1-Hydroxy-1-(4-chlorthiophen-2-yl)-10-(4-acetyl-3-hydroxy-2-propylphenoxy)-deca-3(E),5(Z)-dien-2-ylthio]-4-oxo-4H-1-benzopyran-2-carbonsäure,

7-[(1RS,2SR)-1-Hydroxy-1-(thiophen-2-yl)-10-(4-acetyl-3-hydroxy-2-propyl-phenoxy)-deca-3(E),5(Z)-dien-2-ylthio]-4-oxo-4H-1-benzopyran-2-carbons äure,

7-[(1RS,2SR)-1-Hydroxy-1-(5-fluorthiophen-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1RS,2SR)-1-Hydroxy-1-(5-trifluormethylthiophen-2-yl)-10-(4-acetyl-3-hydroxy-2-propylphenoxy)-deca-3-(E),5(Z)-dien-2-ylthio]-4-oxo-4H-1-benzopyran-2-carbonsäure,

7-[(1RS,2SR)-1-Hydroxy-1-(4-trifluormethylthiophen-2-yl)-10-(4-acetyl-3-hydroxy-2-propylphenoxy)-deca-3-(E),5(Z)-dien-2-ylthio]-4-oxo-4H-1-benzopyran-2-carbonsäure,

7-[(1R,2S)-1-Hydroxy-1-(5-trifluormethylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-methylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-methylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-chlorfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-chlorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-fluorfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-fluorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-methoxycarbonylfur-2-yl)-10-(4-acetyl-3-hydroxy-2-propyl-phenoxy-deca-3(E),5-(Z)-dien-2-ylthio]-4-oxo-4H-1-benzopyran-2-carbonsäure,
7-[(1R,2S)-1-Hydroxy-1-(5-methoxycarbonylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-carboxyfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hyroxy-1-(5-carboxyfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure
7-[(1R,2S)-1-Hydroxy-1-(4-trifluormethylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-methylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(4-methylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-chlorfur-2-yl)-10-(4-acetyl-3-hydroxy-2-propyl-phenoxy-deca-3(E),5(Z)-dien-2-ylthio]-4-oxo-4H-1-benzopyran-2-carbonsäure,
7-[(1R,2S)-1-Hydroxy-1-(4-chlorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-fluorfur-2-yl)-10-(4-acetyl-3-hydroxy-2-propylphenoxy)-deca-3(E),5(Z)-dien-2-ylthio]-4-oxo-4H-1-benzopyran-2-carbonsäure,
7-[(1R,2S)-1-Hydroxy-1-(4-fluorfur-2-yl)-8-(4-acetyl-3-hydroxy-2-propyl-phenoxy-octa-3(E),5(Z)-dien-2-ylthio]-4-oxo-4H-1-benzopyran-2-carbonsäure,
7-[(1R,2S)-1-Hydroxy-1-(4-methoxycarbonylfur-2-yl)-10-(4-acetyl-3-hydroxy-2-propyl-phenoxy)-deca-3(E),5-(Z)-dien-2-ylthio]-4-oxo-4H-1-benzopyran-2-carbonsäure,
7-[(1R,2S)-1-Hydroxy-1-(4-methoxycarbonylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-carboxy-fur-2-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(4-carboxyfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-trifluormethylfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-trifluormethylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-methylfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-methylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-chlorfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-chlorfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-fluorfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-fluorfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-methoxycarbonylfur-3-yl)-10-(4-acetyl-3-hydroxy-2-propyl-phenoxy)-deca-3(E),5-(Z)-dien-2-ylthio]-4-oxo-4H-1-benzopyran-2-carbonsäure,
7-[(1R,2S)-1-Hydroxy-1-(5-methoxycarbonyl-3-yl)-8-(4-acetyl-3-hydroxy-2-propyl-phenoxy)-octa-3(E),5(Z)-dien-2-ylthio]-4-oxo-4H-1-benzopyran-2-carbonsäure
7-[(1R,2S)-1-Hydroxy-1-(5-carboxyfur-3-yl)-10-(4-acetyl-3-hydroxy-2-propylphenoxy)-deca-3(E),5(Z)-dien-2-ylthio]-4-oxo-4H-1-benzopyran-2-carbonsäure oder
7-[(1R,2S)-1-Hydroxy-1-(5-carboxyfur-3-yl)-8-(4-acetyl-3-hydroxy-2-propyl-phenoxy)-octa-3(E),5(Z)-dien-2-ylthio]-4-oxo-4H-1-benzopyran-2-carbonsäure oder jeweils ein Salz davon.

19. Eine Verbindung gemäss einem der Ansprüche 1 bis 4, 9, 10 und 18 zur Anwendung in einem Verfahren zur therapeutischen Behandlung des menschlichen oder tierischen Körpers.

20. Eine Verbindung gemäss einem der Ansprüche 5 bis 8 und 11 bis 17 zur Anwendung in einem Verfahren zur therapeutischen Behandlung des menschlichen oder tierischen Körpers.

21. Pharmazeutische Präparate, als pharmazeutischer Wirkstoff enthaltend eine Verbindung gemäss einem der Ansprüche 1 bis 4, 9, 10, 18 und 19.

22. Pharmazeutische Präparate, als pharmazeutischer Wirkstoff enthaltend eine Verbindung gemäss einem der Ansprüche 5 bis 8, 11 bis 17 und 20.

23. Verfahren zur Herstellung substituierter Alkanophenone der allgemeinen Formel

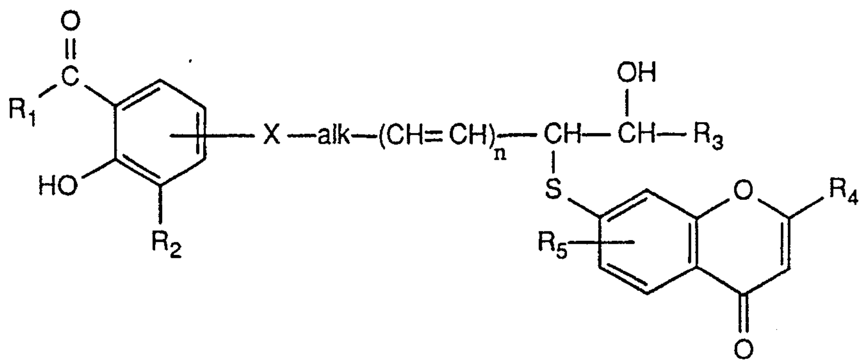

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio oder eine direkte Bindung bedeutet, alk Niederalkylen darstellt, n für 1 oder 2 steht, $R_3$ einen unsubstituierten oder durch gegebenenfalls fluoriertes Niederalkyl, veräthertes oder verestertes Hydroxy, Halogen, gegebenenfalls niederalkyliertes Amino und/oder gegebenenfalls verestertes oder amidiertes Carboxy substituierten 5-gliedrigen, als Heteroatom 1 N-, O- oder S-Atom aufweisenden Heteroarylrest bedeutet, $R_4$ gegebenenfalls verestertes oder amidiertes Carboxy oder 5-Tetrazolyl darstellt und $R_5$ für Wasserstoff oder Niederalkyl steht, und ihre Salze, dadurch gekennzeichnet, dass man ein Epoxid der Formel II

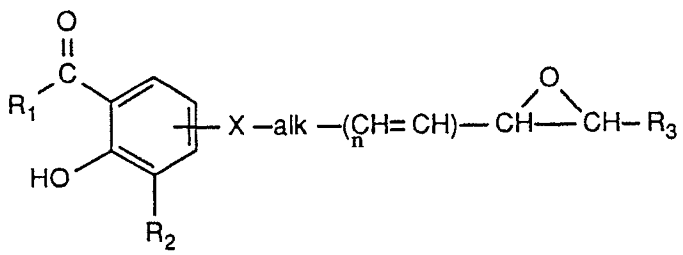

(II).

worin $R_1$, $R_2$, X, alk, n und $R_3$ obige Bedeutungen haben, mit einem Thiol der Formel III

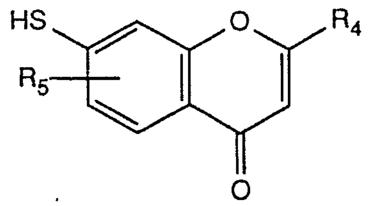

(III),

worin $R_4$ und $R_5$ obige Bedeutungen haben, oder einem Salze davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältliches Salz in die freie Verbindung oder in ein anderes Salz überführt.

24. Verfahren zur Behandlung allergischer Erkrankungen, dadurch gekennzeichnet dass eine Verbindung

gemäss einem der Ansprüche 1 bis 20 bzw. ein pharmazeutisches Präparat gemäss Anspruch 21 oder 22 verabreicht.
25. Verwendung von Verbindungen gemäss einem der Ansprüche 1 bis 20 zur Herstellung eines antiallergischen Arzneimittels.

Patentansprüche für folgende Vertragsstaaten: ES und GR

1. Verfahren zur Herstellung substituierte Alkanophenone der allgemeinen Formel

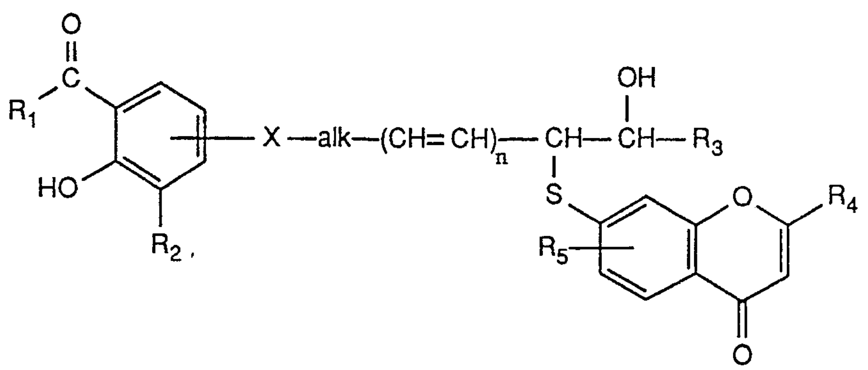

(I),

worin $R_1$ gegebenenfalls fluoriertes Niederalkyl bedeutet, $R_2$ Wasserstoff, gegebenenfalls fluoriertes Niederalkyl oder Niederalkenyl darstellt, X Niederalkylen, Oxy, Thio cder eine direkte Bindung bedeutet, alk Niederalkylen darstellt, n für 1 oder 2 steht, $R_3$ einen unsubstituierten oder durch gegebenenfalls fluoriertes Niederalkyl, veräthertes oder verestertes Hydroxy, Halogen, gegebenenfalls niederalkyliertes Amino und/oder gegebenenfalls verestertes oder amidiertes Carboxy substituierten 5-gliedrigen, als Heteroatom 1 N-, O- oder S-Atom aufweisenden Heteroarylrest bedeutet, $R_4$ gegebenenfalls verestertes oder amidiertes Carboxy oder 5-Tetrazolyl darstellt und $R_5$ für Wasserstoff oder Niederalkyl steht, und ihre Salze, dadurch gekennzeichnet, dass man ein Epoxid der Formel II

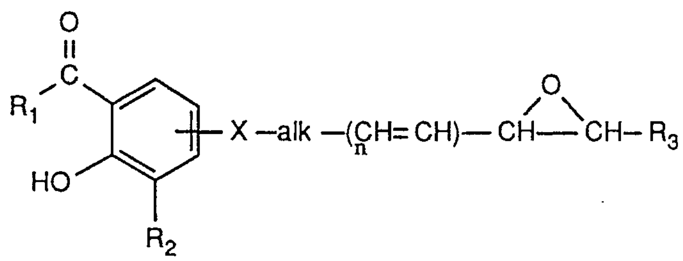

(II).

worin $R_1$, $R_2$, X, alk, n und $R_3$ obige Bedeutungen haben, mit einem Thiol der Formel III

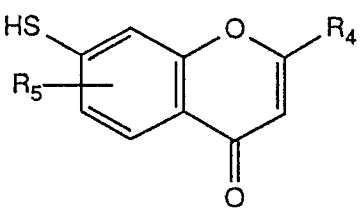

(III),

worin $R_4$ und $R_5$ obige Bedeutungen haben, oder einem Salze davon umsetzt und gewünschtenfalls eine verfahrensgemäss erhältliche Verbindung in eine andere Verbindung der Formel I umwandelt, ein verfahrensgemäss erhältliches Stereoisomerengemisch in die Komponenten auftrennt und/oder eine verfahrensgemäss erhältliche freie Verbindung in ein Salz oder ein verfahrensgemäss erhältiches Salz in die freie Verbindung oder in ein anderes Salz überführt.
2. Verfahren gemäss Anspruch 1 zur Herstellung von Verbindungen der Formel I, worin $R_1$ Niederalkyl oder Mono-, Di- oder Polyfluorniederalkyl bedeutet, $R_2$ Wasserstoff, Niederalkyl, Niederalkenyl oder Mono-, Di- oder Polyfluorniederalkyl darstellt, X Niederalkylen, Oxy oder Thio darstellt alk Niederalkylen bedeutet, $R_3$

unsubstituiertes oder durch Niederalkyl, Niederalkoxy, Halogen, Carboxy, Niederalkoxycarbonyl, Amino, N-Mono- oder N,N-Diniederalkylamino, Carbamyl, N-Mono-oder N,N-Diniederalkylcarbamyl und/oder Trifluormethyl substituiertes Pyrryl, Thienyl oder Furyl bedeutet, $R_4$ Carboxy, Niederalkoxycarbonyl, 5-Tetrazolyl, Carbamyl, N-Mono- oder N,N-Diniederalkylcarbamyl oder gegebenenfalls im Phenylteil durch Niederalkyl, Niederalkoxy, Halogen und/oder Trifluormethyl substituiertes N-(Benzolsulphonyl)carbamoyl bedeutet und $R_5$ für Wasserstoff oder Niederalkyl steht, und ihre Salze.

3. Verfahren gemäss Anspruch 1 zur Herstellung von Verbindungen der Formel I, worin $R_1$ $C_1$-$C_4$-Alkyl oder ω,ω,ω-Trifluor-$C_1$-$C_4$-alkyl bedeutet, $R_2$ Wasserstoff, $C_1$-$C_4$-Alkyl, $C_2$-$C_4$-Alkenyl oder ω,ω,ω-Trifluor-$C_1$-$C_4$-alkyl darstellt, X $C_1$-$C_3$-Alkylen, Oxy oder Thio darstellt, alk geradkettiges $C_2$-$C_6$-Alkylen darstellt n für 1 oder 2 steht, $R_3$ unsubstituiertes oder durch $C_1$-$C_4$-Alkyl, $C_1$-$C_4$-Alkoxy, Halogen der Atomnummer bis und mit 35, Trifluormethyl, Carboxy und/oder $C_1$-$C_4$-Alkoxycarbonyl, substituiertes Pyrryl, Thienyl oder Furyl bedeutet, $R_4$ Carboxy oder N-(Benzolsulphonyl)carbamyl bedeutet, und $R_5$ für Wasserstoff steht, und ihre Salze.

4. Verfahren gemäss Anspruch 1 zur Herstellung von Verbindungen der Formel

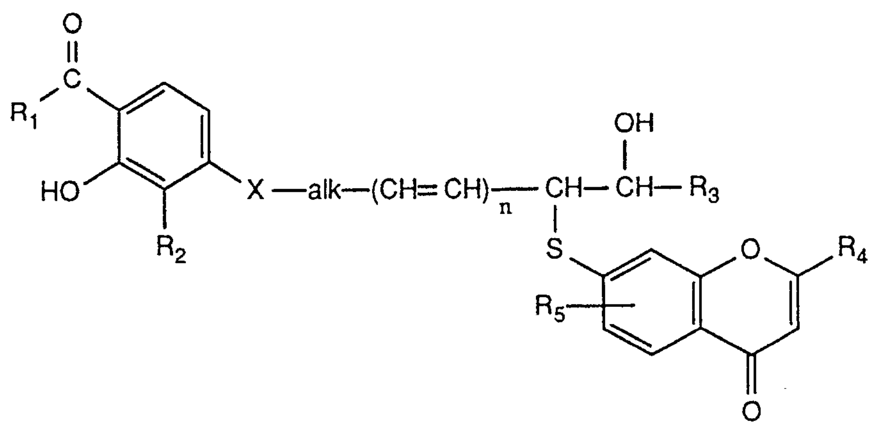

(Ia),

worin $R_1$ $C_1$-$C_4$-Alkyl, bedeutet, $R_2$ $C_1$-$C_4$-Alkyl darstellt, X für Oxy steht, alk $C_2$-$C_6$-Alkylen darstellt, n für 1 oder 2 steht, $R_3$ unsubstituiertes oder durch $C_1$-$C_4$-Alkyl, $C_1$-$C_4$-Alkoxy, Halogen der Atomnummer bis und mit 35, Trifluormethyl oder $C_1$-$C_4$-Alkoxycarbonyl substituiertes Pyrryl, Thienyl oder Furyl bedeutet, $R_4$ Carboxy bedeutet, und $R_5$ Wasserstoff ist, und ihre Salze.

5. Verfahren gemäss Anspruch 4 zur Herstellung von Verbindungen der Formel Ia, worin $R_1$ $C_1$-$C_4$-Alkyl bedeutet, $R_2$ $C_1$-$C_4$-Alkyl darstellt, X für Oxy steht, alk $C_2$-$C_6$-Alkylen darstellt, n für 2 steht, $R_3$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Thienyl oder Furyl bedeutet, $R_4$ Carboxy bedeutet und $R_5$ Wasserstoff ist, und ihre Salze.

6. Verfahren gemäss Anspruch 4 zur Herstellung von Verbindungen der Formel Ia, worin $R_1$ $C_1$-$C_4$-Alkyl bedeutet, $R_2$ $C_1$-$C_4$-Alkyl darstellt, X für Oxy steht, alk $C_2$-$C_5$-Alkylen darstellt, n für 2 steht, $R_3$ unsubstituiertes oder durch Halogen der Atomnummer bis und mit 35 mono- oder disubstituiertes 3-Thienyl oder 3-Furyl bedeutet, $R_4$ Carboxy bedeutet und $R_5$ Wasserstoff ist, und ihre Salze.

7. Verfahren gemäss Anspruch einem der Ansprüche 1 bis 6 zur Herstellung von Verbindungen der Formel I bzw. Ia, worin die mit dem Rest alk verbundene Doppelbindung in (Z)-, d.h. cis-Konfiguration und die gegebenenfalls vorhandene zusätzliche Doppelbindung in (E)-, d.h. trans-Konfiguration vorliegt.

8. Verfahren gemäss Anspruch einem der Ansprüche 1 bis 7 zur Herstellung von Verbindungen der Formel I bzw. Ia, worin das mit dem Schwefelatom verbundene Ketten-C-Atom (S)- und das die Hydroxygruppe tragende Ketten-C-Atom (R)-Konfiguration besitzt.

9. Verfahren gemäss Anspruch 1 zur Herstellung von 7-[(1RS,2SR)-1-Hydroxy-1-(thiophen-3-yl)-10-(4-acetyl-3-hydroxy-2-propyl-phenoxy)-deca-3(E),5(Z)-dien-2-ylthio]-4-oxo-4H-1-benzopyran-2-carbonsäuremethylester.

10. Verfahren gemäss Anspruch 1 zur Herstellung von 7-[(1RS,2SR)-1-Hydroxy-1-(thiophen-3-yl)-10-(4-acetyl-3-hydroxy-2-propyl-phenoxy)-deca-3(E),5(Z)-dien-2-ylthio]-4-oxo-4H-1-benzopyran-2-carbonsäure oder eines ihrer Salze.

11. Verfahren gemäss Anspruch 1 zur Herstellung von 7-[(1RS,2SR)-1-Hydroxy-1-(thiophen-3-yl)-8-(4-acetyl-3-hydroxy-2-propylphenoxy)-octa-3(E),5(Z)-dien-2-ylthio]-4-oxo-4H-1-benzopyran-2-carbonsäuremethylester

12. Verfahren gemäss Anspruch 1 zur Herstellung von 7-[(1RS,2SR)-1-Hydroxy-1-(thiophen-3-yl)-8-(4-acetyl-3-hydroxy-2-propylphenoxy)-octa-3(E),5(Z)-dien-2-ylthio]-4-oxo-4H-1-benzopyran-2-carbonsäure oder eines ihrer Salze.

13. Verfahren gemäss Anspruch 1 zur Herstellung von7-[(1RS,2SR)-Hydroxy-1-(fur-3-yl)-10-(4-acetyl-3-hydroxy-2-propylphenoxy)-deca-3(E),5(Z)-dien-2-ylthio]-4-oxo-4H-1-benzopyran-2-carbonsäure oder eines ihrer Salze.

14. Verfahren gemäss Anspruch 1 zur Herstellung von 7-[(1RS,2SR)-Hydroxy-1-(fur-3-yl)-9-(4-acetyl-3-hydroxy-2-propylphenoxy)-nona-3(E),5(Z)-dien-2-ylthio]-4-oxo-4H-1-benzopyran-2-carbonsäure oder eines ihrer Salze.

15. Verfahren gemäss Anspruch 1 zur Herstellung von 7-[(1RS,2SR)-Hydroxy-1-(fur-3-yl)-8-(4-acetyl-3-hydroxy-2-propylphenoxy)-octa-3(E),5(Z)-dien-2-ylthio]-4-oxo-4H-1-benzopyran-2-carbonsäure oder eines ihrer Salze.

16. Verfahren gemäss Anspruch 1 zur Herstellung von 7-[(1RS,2SR)-Hydroxy-1-(2,5-dichlorthiophen-3-yl)-10-(4-acetyl-3-hydroxy-2-propylphenoxy)-deca-3(E),5(Z)- dien-2-ylthio]-4-oxo-4H-1-benzopyran-2-carbonsäuremethylester.

17. Verfahren gemäss Anspruch 1 zur Herstellung von 7-[(1RS,2SR)-Hydroxy-1-(2,5-dichlorthiophen-3-yl)-10-(4-acetyl-3-hydroxy-2-propyl-phenoxy)-deca-3(E),5(Z)-dien-2-ylthio]-4-oxo-4H-1-benzopyran-2-carbonsäure oder eines ihrer Salze.

18. Verfahren gemäss Anspruch 1 zur Herstellung von

7-[(1R,2S)-1-Hydroxy-1-(5-chlorthiophen-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-trifluormethylfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1RS,2SR)-1-Hydroxy-1-(4-chlorthiophen-2-yl)-10-(4-acetyl-3-hydroxy-2-propylphenoxy)-deca-3(E),5(Z)-dien-2-ylthio]-4-oxo-4H-1-benzopyran-2-carbonsäure,

7-[(1RS,2SR)-1-Hydroxy-1-(thiophen-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1RS,2SR)-1-Hydroxy-1-(5-fluorthiophen-2-yl)-10-(4-acetyl-3-hydroxy-2-prcpyl-phenoxy)-deca-3(E),5(Z)-dien-2-ylthio]-4-oxo-4H-1-benzopyran-2-carbonsäure,

7-[(1RS,2SR)-1-Hydroxy-1-(5-trifluormethylthiophen-2-yl)-10-(4-acetyl-3-hydroxy-2-propylphenoxy)-deca-3-(E),5(Z)-dien-2-ylthio]-4-oxo-4H-1-benzopyran-2-carbonsäure,

7-[(1RS,2SR)-1-Hydroxy-1-(4-trifluormethylthiophen-2-yl)-10-(4-acetyl-3-hydroxy-2-propylphenoxy)-deca-3-(E),5(Z)-dien-2-ylthio]-4-oxo-4H-1-benzopyran-2-carbonsäure,

7-[(1R,2S)-1-Hydroxy-1-(5-trifluormethylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-methylfur-2-yl)-10-(4-acetyl-3-hydroxy-2-propyl-phenoxy)- deca-3(E),5(Z)-dien-2-ylthio]-4-oxo-4H-1-benzopyran-2-carbonsäure,

7-[(1R,2S)-1-Hydroxy-1-(5-methylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-chlorfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-chlorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-fluorfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-fluorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-methoxycarbonylfur-2-yl)-10-(4-acetyl-3-hydroxy-2-propyl-phenoxy-deca-3(E),5-(Z)-dien-2-ylthio]-4-oxo-4H-1-benzopyran-2-carbonsäure,

7-[(1R,2S)-1-Hydroxy-1-(5-methoxycarbonylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(5-carboxyfur-2-yl)-10-(4-acetyl-3-hydroxy-2-propyl-phenoxy)-deca-3(E),5(Z)-dien-2-ylthio]-4-oxo-4H-1-benzopyran-2-carbonsäure,

7-[(1R,2S)-1-Hydroxy-1-(5-carboxyfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,

7-[(1R,2S)-1-Hydroxy-1-(4-trifluormethylfur-2-yl)-10-(4-acetyl-3-hydroxy-2-propyl-phenoxy)-deca-3(E),5(Z)-

dien-2-ylthio]-4-oxo-4H-1-benzopyran-2-carbonsäure
7-[(1R,2S)-1-Hydroxy-1-(4-trifluormethylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-methylfur-2-yl)-10-(4-acetyl-3-hydroxy-2-propyl-phenoxy)- deca-3(E),5(Z)-dien-2-ylthio]-4-oxo-4H-1-benzopyran-2-carbonsäure,
7-[(1R,2S)-1-Hydroxy-1-(4-methylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-chlorfur-2-yl)-10-(4-acetyl-3-hydroxy-2-propyl-phenoxy-deca-3(E),5(Z)-dien-2-ylthio]-4-oxo-4H-1-benzopyran-2-carbonsäure,
7-[(1R,2S)-1-Hydroxy-1-(4-chlorfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-fluorfur-2-yl)-10-(4-acetyl-3-hydroxy-2-propylphenoxy)-deca-3(E),5(Z)-dien-2-ylthio]-4-oxo-4H-1-benzopyran-2-carbonsäure,
7-[(1R,2S)-1-Hydroxy-1-(4-fluorfur-2-yl)-8-(4-acetyl-3-hydroxy-2-propyl-phenoxy-octa-3(E),5(Z)-dien-2-ylthio]-4-oxo-4H-1-benzopyran-2-carbonsäure,
7-[(1R,2S)-1-Hydroxy-1-(4-methoxycarbonylfur-2-yl)-10-(4-acetyl-3-hydroxy-2-propyl-phenoxy)-deca-3(E),5-(Z)-dien-2-ylthio]-4-oxo-4H-1-benzopyran-2-carbonsäure,
7-[(1R,2S)-1-Hydroxy-1-(4-methoxycarbonylfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(4-carboxy-fur-2-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(4-carboxyfur-2-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-trifluormethylfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-trifluormethylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-methylfur-3-yl)-10-(4-acetyl-3-hydroxy-2-propyl-phenoxy)- deca-3(E),5(Z)-dien-2-ylthio]-4-oxo-4H-1-benzopyran-2-carbonsäure,
7-[(1R,2S)-1-Hydroxy-1-(5-methylfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-chlorfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-chlorfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-fluorfur-3-yl)-10-(4-acetyl-3-hydroxy-2-propyl-phenoxy)-deca-3(E),5(Z)-dien-2-ylthio]-4-oxo-4H-1-benzopyran-2-carbonsäure,
7-[(1R,2S)-1-Hydroxy-1-(5-fluorfur-3-yl)-8-(4-acetyl-3-hydroxy-2-propyl-phenoxy)-octa-3(E),5(Z)-dien-2-ylthio]-4-oxo-4H-1-benzopyran-2-carbonsäure,
7-[(1R,2S)-1-Hydroxy-1-(5-methoxycarbonylfur-3-yl)-10-(4-acetyl-3-hydroxy-2-propyl-phenoxy)-deca-3(E),5-(Z)-dien-2-ylthio]-4-oxo-4H-1-benzopyran-2-carbonsäure,
7-[(1R,2S)-1-Hydroxy-1-(5-methoxycarbonyl-3-yl)-8-(4-acetyl-3-hydroxy-2-propyl-phenoxy)-octa-3(E),5(Z)-dien-2-ylthio]-4-oxo-4H-1-benzopyran-2-carbonsäure
7-[(1R,2S)-1-Hydroxy-1-(5-carboxyfur-3-yl)-10-(4-acetyl-3-hydroxy-2-propylphenoxy)-deca-3(E),5(Z)-dien-2-ylthio]-4-oxo-4H-1-benzopyran-2-carbonsäure oder
7-[(1R,2S)-1-Hydroxy-1-(5-carboxyfur-3-yl)-8-(4-acetyl-3-hydroxy-2-propyl-phenoxy)-octa-3(E),5(Z)-dien-2-ylthio]-4-oxo-4H-1-benzopyran-2-carbonsäure oder jeweils eines Salzes davon.

19. Verfahren zur Herstellung pharmazeutischer Präparate, dadurch gekennzeichnet, dass man eine gemäss einem der Ansprüche 1 bis 18 erhältliche Verbindung mit üblichen pharmazeutischen Hilfsstoffen vermischt.